# EUROPEAN PATENT APPLICATION

(11) **EP 4 813 386 A1**
(43) Date of publication of application: **30.09.2026**
(21) Application number: 25382296.9
(22) Date of filing: 27.03.2025
(51) Int. Cl.: A61K 31/4439, A61K 38/06, A61K 45/06, A61P 25/00, A61P 43/00

(54) **HDAC6 INHIBITORS FOR THE TREATMENT OF RETT SYNDROME**

(71) Applicant: QUIMATRYX, S.L., 20009 San Sebastián, Guipuzcoa (ES)
(72) Inventor: The designation of the inventor has not yet been filed
(74) Representative: Mewburn Ellis LLP

(57) **Abstract**

The present invention relates to a
compound (HDAC6 inhibitor) for use in the treatment of Rett syndrome (RTT), wherein the compound has a formula according to formula (A): 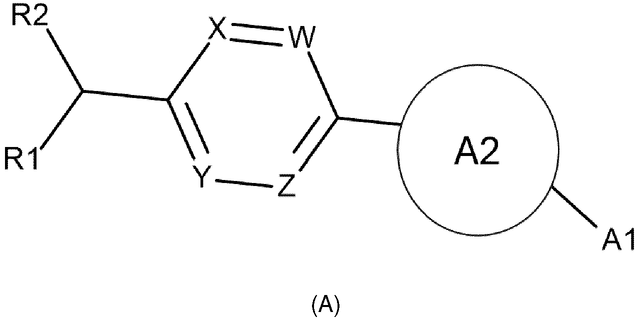 or a salt, solvate, stereoisomer or prodrug thereof,
wherein
A1 is CF₃, CHF₂, CH₂F
A2 is an oxadiazole ring;
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (A).

## Description

### FIELD OF THE INVENTION

This invention generally pertains to the field of pharmaceuticals and pharmacy and specifically to pharmaceutical formulations comprising a certain compound, which is a histone deacetylase inhibitor (HDAC6 inhibitor). In particular, the present invention pertains to the use of the said compound in the treatment of Rett syndrome (RTT).

### BACKGROUND

### Rett syndrome (RTT)

Rett syndrome (RTT) is a debilitating neurological disorder in girls, which is caused by a mutation on the X chromosome. It leads to the development of motor and neurological disfunction and is estimated to affect 1 in every 10,000 live female births.

Girls affected with RTT usually develop symptoms between 6 months and 2 years of age, which may include loss of speech and motor skills, difficulty walking, seizures, gait abnormalities, repetitive hand movements and irregular breathing patterns.

Fewer than 1% of cases of RTT are believed to be inherited from parents and RTT is found in all racial and ethnic groups. More than 95% of RTT patients carry a mutation in the MECP2 gene. MECP2 binds to methylated DNA to regulate gene transcription through repression or activation. The genetic loss of MECP2 has been identified as changing the properties of cells in the locus ceruleus (LC) the exclusive source of noradrenergic innervation to the cerebral cortex and hippocampus. These neurons are a pivotal source of norepinephrine throughout the brainstem and forebrain and are involved in the regulation of diverse functions disrupted in Rett syndrome, such as respiration and cognition. The locus ceruleus is consequently a critical site at which loss of MECP2 results in CNS disfunction.

Despite the genetic cause being known in the majority of cases, the pathophysiology of the neurological phenotype of RTT is largely unknown. Tubulin and the microtubule network play an essential role in neuronal function, with the acetylation state of microtubules dictating the efficiency of neuronal migration and differentiation, synaptic targeting and molecular motor trafficking of mRNA, high energy mitochondria and brain-derived neurotrophic factor (BDNF)-containing vesicles (cf. W A Gold et al. J. Mol. Med (Berl) 2015 Jan;93(1):63-72). Perturbations in tubulin and microtubule dynamics have been shown in MeCP2-dificient cells, suggesting a link between the aberrations and the neurobiology of RTT.

Currently, there is no effective treatment for RTT and the condition is largely managed by treating the symptoms of this disease as they arise. It is, therefore, highly desirable to develop a treatment which is effective against the causes of RTT.

### Histone Deacetylase (HDAC)

Histone deacetylases (HDAC) constitute an interesting therapeutic target for the treatment of diseases related to the central nervous system, such as autoimmune diseases (cf. A. G. Kazantsev, L. M. Thompson Nature Rev. Drug Discov. 2006, 7, 854), Alzheimer's disease (cf. US2019135799 A1), supranuclear palsy (cf. WO2021060567 (A1)), peripheral neuropathy, stroke (cf. US2020039933 (A1)) etc.

Several families of HDAC inhibitors (HDACis) have been designed, whose general structures can be found in different reviews (cf. A. Villar-Garea, M. Esteller Int. J. Cancer 2004, 112, 171; T. A. Miller et al. J. Med. Chem. 2003, 46, 5097; T. Suzuki, N. Miyata Curr. Med. Chem. 2005, 12, 2867; M. Paris et al. J. Med. Chem. 2008, 51, 1505). The general structure of these inhibitors consists of a cyclic structure, a spacer and a chelating group capable of binding to the Zn (II) cation of the active centre of the different HDAC isoforms that belong to the class I (HDAC1, HDAC2, HDAC3 and HDAC8), class II (HDAC4, HDAC5, HDAC6, HDAC7, HDAC9 and HDAC10) and class IV (HDAC11).

The mechanism of action of the HDAC inhibitors is explained by their antagonist properties against histone deacetylases. It has been shown that inhibition of HDAC6 can restore tubulin acetylation levels (cf. W A Gold et al. J. Mol. Med (Berl) 2015 Jan;93(1):63-72), which may lead to a reduction in the symptoms of RTT.

One important class of HDAC inhibitors are 1,3,4,-oxadiazole derivatives, are described in, for example, WO2020212479.

### SUMMARY OF THE INVENTION

In view of the practical problem outlined above, the present inventors have developed a pharmaceutical treatment for RTT which involves administering a HDAC6 inhibitor to a patient. The HDAC6 inhibitors according to the present invention are non-hydroxamate HDAC6 inhibitors.

Accordingly, in a first aspect the present invention provides a compound (HDAC6 inhibitor) for use in the treatment of Rett syndrome (RTT), wherein the compound has a formula according to formula (A): 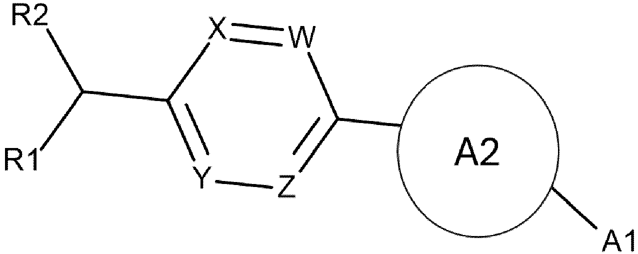 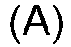 or a salt, solvate, stereoisomer or prodrug thereof,
wherein
A1 is CF₃, CHF_{2,} CH₂F
A2 is an oxadiazole ring;
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (A).

Preferably, the present invention relates to a compound (HDAC6 inhibitor) for use in the treatment of RTT, wherein the compound has a formula according to formula (V):

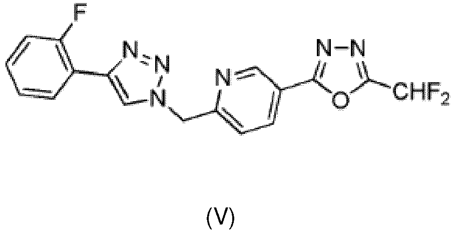

Alternatively, the present invention relates to a compound (HDAC6 inhibitor) for use in the treatment of RTT, wherein the compound has a formula according to formula (VI):

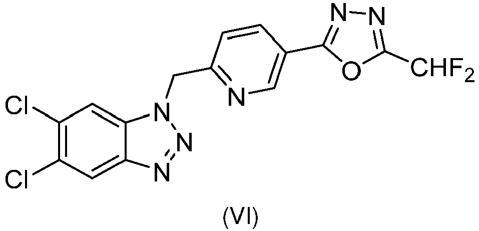

Another aspect of the present invention relates to a pharmaceutical composition comprising a compound according to formula (A) and at least one excipient for use in the treatment of RTT.

Another aspect of the invention relates to pharmaceutical composition comprising a compound (HDAC6 inhibitor) according to formula (A) for use in the treatment of RTT, wherein the compound according to formula (A) is administered simultaneously or sequentially with a second pharmaceutical agent.

Another aspect of the present invention relates to the use of a compound (HDAC6 inhibitor) of formula (A) or salt, solvate, stereoisomer or prodrug thereof, in the manufacture of a medicament for use in a method of treating RTT.

According to another aspect, the present invention relates to a method of treating an animal having or suspected of having RTT which comprises administering to a subject needing such treatment, a therapeutically effective amount of a compound (HDAC6 inhibitor) of formula (A) or salt, solvate, stereoisomer or prodrug thereof.

As will be appreciated by one of skill in the art, features and preferred embodiments of one aspect of the invention also pertain to other aspects of the invention.

Firstly, the compounds (HDAC6 inhibitors) according to formula (A) above are effective in treating RTT in both *in vitro* and *in vivo* models. Without being bound by any theory, it is believed that compounds with the structures set out above have improved efficacy in treating RTT compared to other HDAC6 inhibitors.

Secondly, the compounds according to formula (A) are generally well tolerated and exhibit much lower levels of toxicity in vitro compared with other HDAC6 inhibitors, meaning that larger doses of the compounds according to formula (A) can be used.

Thirdly, the compounds according to formula (A) generally show good blood brain barrier penetration compared to other HDAC6 inhibitors such as ACY1215. This is in particular the case for compounds according to formula (V). This leads to high concentrations of the drug in the brain compared to other HDAC6 inhibitors.

Without being bound by any theory it is believed that the fact that the compounds according to the present invention are non-hydroxamic HDAC6 inhibitors leads to superior blood brain barrier penetration. Good blood-brain barrier penetration is considered necessary for the effective treatment of RTT.

Fourthly, the compounds according to formula (A) are effective in treating the symptoms of RTT such as tremor development and reduced mobility. The compounds according to formula (A) may also be effective in helping a subject to maintain or retain balance. Prior disclosures have evaluated potential RTT therapeutics primarily in simple *in vitro* models, which lack the sophistication necessary to evaluate improved mobility and/or reduction in tremor.

Fifthly, the compounds according to formula (A) have high bioavailability compared to other HDAC6 inhibitors.

For at least the reasons given above, the present invention represents a promising advancement in the treatment of RTT.

### BRIEF DESCRIPTION OF THE FIGURES

Embodiments and experiments illustrating the principles of the invention will now be discussed with reference to the accompanying figures:
**Figure 1** shows a composite graph showing the concentration of each compound against cell viability.
**Figure 2** shows a Western blot showing acetylated α-tubulin levels in MECP2-KO cells after 48 hours of treatment with each compound. Mock-treated (DMSO-treated) cells are shown for comparison. Total α-tubulin was measured as a loading control, along with β-actin.
**Figure 3** shows a Western blot showing acetylation levels measured 48 hours after treatment with the compound, along with HDAC6 levels (which are not expected to change). Mock-treated (DMSO-treated) cells are shown for comparison. Total α-tubulin was measured as a loading control, along with β-actin.
**Figure 4** shows a Western blot showing acetylation levels after 48 hours of treatment with HDAC6 inhibitors QTX166 (400 nM) and QTX153 (200 nM). *MECP2*-KO cells are analyzed in the upper panels, and MeCP2 point mutation R133C is analyzed in the lower panels. Mock-treated (DMSO-treated) cells are shown for comparison. Total α-tubulin was measured as a loading control, along with β-actin.
**Figure 5** shows a graph showing the diameter of neurosphere in *MECP2*-KO neural progenitor cells following treatment with various with HDAC6 inhibitors and Trofinetide.
**Figure 6** shows an image constructed using NeuroStudio analysis of whole neurons in culture.
**Figure 7** shows an image constructed using Sholl analysis to estimate the number of branches present on the neuron.
**Figure 8** shows the total number of branch points for *MECP2*-KO Neural Progenitors Under Glutamatergic Differentiation.
**Figure 9** shows the total number of branch points for *MECP2-*R133C Neural Progenitors Under Glutamatergic Differentiation.
**Figure 10 Figure 10A** shows the body weight of *MECP2*-KO Male Mice, *MECP2*-KO Male Mice treated with vehicle and *MECP2*-KO Male Mice treated with vehicle and QTX153. **Figure 10B** shows the precentage change in body weight for *MECP2*-KO Male Mice, *MECP2*-KO Male Mice treated with vehicle and *MECP2*-KO Male Mice treated with vehicle and QTX153.
**Figure 11** shows survival curves for *MECP2*-KO Male Mice, *MECP2*-KO Male Mice treated with vehicle and *MECP2*-KO Male Mice treated with vehicle and QTX153.
**Figure 12** shows bar charts corresponding to Male Mecp2-KO Mouse Model of Rett Syndrome treated with vehicle or QTX153, who were regularly scored for the symptoms indicated on the graphs. The bars represent values corresponding to mice of the same age.
**Figure 13** shows a Western blot to detect markers of treatment in the cerebellum. Western blot analysis of cerebellar extracts from experimental mice at end-point, where α-tubulin and its acetylation levels are analysed. Graphs on the right represent quantitation of band intensity (unpaired t test, * p < 0.05).
**Figure 14** shows a Western blot to detect markers of treatment in various brain regions. Western blot analysis of acetyl-α-tubulin, total α-tubulin and β-actin in total protein extracts from the hippocampus (A), and thalamus (B) of control-treated or QTX153-treated animals at the endpoint. WT animals of similar ages are also evaluated for comparion. Graphs represent quantitation of band intensity.
**Figure 15** shows a Western blot analysis of Synapsin-1 and β-actin in total protein extracts from the frontal cortex (A), hippocampus (B), olfactory bulb (C) and pons (D). Graphs represent quantitation of band intensity (unpaired t test, * p < 0.05, ** p < 0.01, ns = not significant).
**Figure 16** shows a Western blot analysis of PSD95 and β-actin in total protein extracts from the cerebellum. Graphs represent quantitation of band intensity (unpaired t test, * p < 0.05).
**Figure 17** shows an Immunostaining analysis of Psd95 in the cerebellum. Immunostaining analysis of PSD95 (red) in the cerebellum of sagittal sections counterstained with DAPI (blue). All images were processed with ImageJ Fiji version v1.53k.
**Figure 18** shows an Immunostaining analysis of VGlut1 (red) in the cerebellum of sagittal sections counterstained with DAPI (blue). All images were processed with ImageJ Fiji version v1.53k.

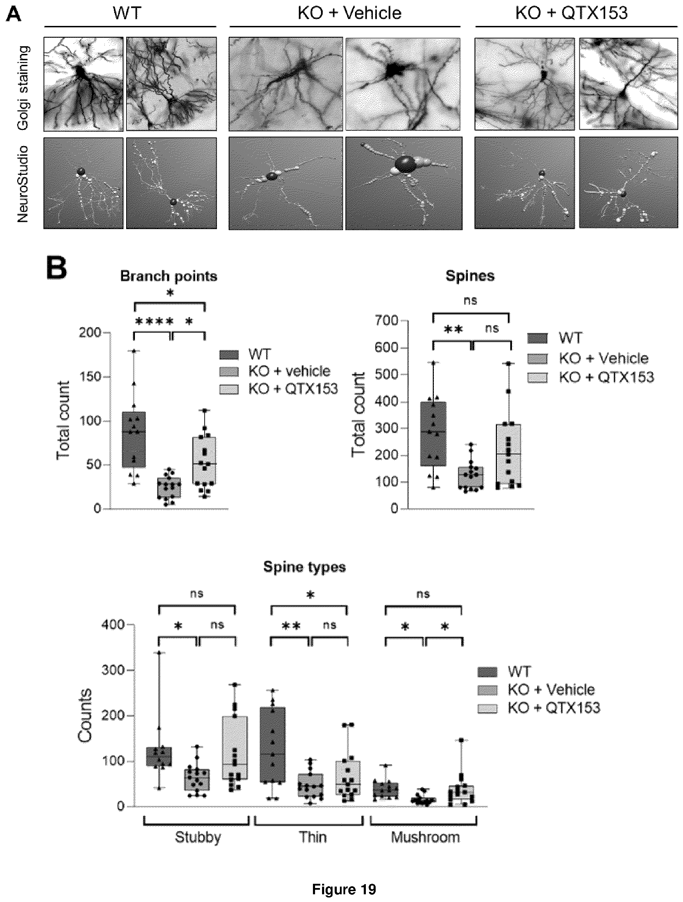

**Figure 19** shows a morphological analysis of hippocampal neurons. (A) Two representative pictures of hippocampal sections from WT, vehicle-treated, or QTX153-treated mice are shown. The images depict Golgi staining (above) and digital reconstructions using the NeuroStudio software (below). (B) Automatic counting of the number of branches, total number of spines, or specific spine subtypes. Graphs represent the average number of branch points or spines ± SEM. n = 15 for Mecp2-KO animals, n = 13 for WT samples. One-way ANOVA test was used (*p < 0.05, **p < 0.01, ****p < 0.0001, ns = not significant).
**Figure 20** shows a Morphological analysis of cerebellar neurons. (A) Two representative pictures of sections from the cerebellum of vehicle-treated or QTX153-treated mice are shown. The images depict Golgi staining (above) and digital reconstructions using the NeuroStudio software (below). (B) Automatic counting of the number of branches, total number of spines, or specific spine subtypes. Graphs represent the average number of branch points or spines ± SEM. (n = 15, One-way ANOVA test was used, *p < 0.05, **p < 0.01, *** p < 0.001, ns = not significant).
**Figure 21** shows Morphological analysis of cortical neurons. (A) Two representative pictures of sections from the cortex of vehicle-treated or QTX153-treated mice are shown. The images depict Golgi staining (above) and digital reconstructions using the NeuroStudio software (below). (B) Automatic counting of the number of branches, total number of spines, or specific spine subtypes. Graphs represent the average number of branch points or spines ± SEM. (n = 13, One-way ANOVA test was used, *p < 0.05, ns = not significant).

### DETAILED DESCRIPTION

### Definitions

Unless otherwise defined, all technical and scientific terms used herein have the same meaning as commonly understood by one of ordinary skill in the art to which this disclosure belongs. Methods and materials are described herein for use in the present disclosure; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

The term "about" preceding a stated value indicates that the value may have an uncertainty of ± 20%, preferably ± 10%, ± 5%, ± 2%, ± 1% of the stated value.

The term "room temperature" refers to the ambient temperature of a typical laboratory, which is typically between 20 ºC and 30 ºC, preferably around 25 ºC, at atmospheric pressure.

The term "injection" refers to any form of injection known to a skilled person in the art such as subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal. Injection may refer to an infusion process (e.g. sustained administration) as well as bolus (discreate) administration.

The term "treatment" or "treating" refers to administration of a compound or a pharmaceutical composition of the invention to improve or eliminate the disease or one or more symptoms associated with the disease. The term "prevention" or "prevent" includes reducing the risk of the disease appearing or developing.

Unless otherwise stated, halogen (halo) is selected from chloro (Cl), fluoro (F), bromo (Br) and iodo (I), such as fluoro or chloro. That is the term halogen refers to a chlorine (Cl), Fluorine (FI), Bromine (Br) or Iodine (I).

Hydroxy: -OH.

The compounds of formula (A) may be in the form of salts, solvates, stereoisomers or prodrugs.

It may be convenient or desirable to prepare, purify, and/or handle a corresponding salt of the compounds of formula (A), for example, a pharmaceutically-acceptable salt. Examples of pharmaceutically acceptable salts are discussed in *Berge 1977.*

For example, if the compound is anionic, or has a functional group which may be anionic (e.g. -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of suitable organic cations include, but are not limited to, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g. -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, trifluoroacetic acid and valeric. Examples of suitable polymeric organic anions include, but are not limited to, those derived from the following polymeric acids: tannic acid, carboxymethyl cellulose.

The term "solvate" in accordance with this invention should be understood as meaning any compound of formula (A) according to the present invention in which said compound is bonded by a non-covalent bond to another molecule (normally a polar solvent), including especially hydrates and alcoholates such as methanolate. A preferred solvate is the hydrate. Preferably, the solvate is a pharmaceutically acceptable solvate, i.e. a solvate that is tolerated physiologically, meaning that it is not toxic, particularly, as a result of the solvating molecule when used in an appropriate manner (i.e. in reasonable medical doses) for a treatment according to the present invention.

As used herein, the term "stereoisomer" refers to enantiomers, diastereomers, or mixtures thereof, such as a racemates, of the compounds of formula (A) according to the present invention. For example, in formula (A) a chiral centre may occur, for example, at the carbon bonded to R1 and R2 resulting in the possibility of two stereoisomers. Likewise, the term "stereoisomer" also encompasses geometric isomers about any double bonds present in the compound of formula (A), i.e. (E)-isomers and (Z)-isomers (trans and cis isomers). Furthermore, the term also embraces rotamers of the compounds of formula (A).

Any compound of formula (A) according to the present invention may exist in different tautomeric forms. Specifically, the term "tautomer" refers to one of two or more structural isomers of a compound of formula (A) that exist in equilibrium and are readily converted from one isomeric form to the other. Common tautomeric pairs are amine-imine, amide-imidic acid, or keto-enol.

The term "prodrug" refers to derivatives of the compounds of formula (A) that are converted in vivo into the compounds of formula (A), such as by enzymatic hydrolysis.

The term "oxadiazole ring" refers to a class of heterocyclic aromatic chemical compounds with the molecular formula C₂H₂N₂O. For example, 1,2,4-oxadiazole, 1,2,5-oxadiazole, and 1,3,4-oxadiazole. Generally, the oxadiazole ring is attached to other parts of a molecule through bonds at the carbon atoms.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known. The term "substituted" may refer to a parent group substituted with a C₁₋₆ alkyl, OH, - NH₂, aryl, heterocyclyl, C₁₋₆ alkoxy, -NH-( C₁₋₆ alkyl), halogen.

### Alkyl

The term "alkyl" refers to a saturated linear or branched hydrocarbon group, preferably comprising 1 to 20 carbon atoms. The term "alkyl" is used to refer both to monovalent hydrocarbon groups (commonly known as "alkyl" groups) and to divalent hydrocarbon groups (commonly known as "alkylene" groups), which may be obtained by removing one or more hydrogen atoms from a carbon atom of a hydrocarbon compound, which is saturated and may also be branched. The terms "alkyl" and "alkylene" are used interchangeably herein. Preferably, an "alkyl" is a C₁₋₆ alkyl having from 1 to 6 carbon atoms or a C₁₋₄ alkyl having from 1 to 4 carbon atoms and which are saturated.

Examples of alkyl groups include, but are not limited to, methyl (C1), ethyl (C2), propyl (C3), butyl (C4), pentyl (C5) and hexyl (C6).

Alkyl groups may be linear or branched.

Examples of linear alkyl groups include, but are not limited to, methyl (C1), ethyl (C2), n-propyl (C3), n-butyl (C4), n-pentyl (amyl) (C5) and n-hexyl (C6).

Examples of branched alkyl groups include iso-propyl (C3), iso-butyl (C4), sec-butyl (C4), tert-butyl (C4), iso-pentyl (C5), and neo-pentyl (C5).

Alkyl groups may be substituted or unsubstituted. Within the meaning of this invention unless explicitly defined in the formulae the alkyl groups are preferably unsubstituted.

### Aryl

The term "aryl" refers to cyclic, aromatic hydrocarbon groups that have 1 to 2 aromatic rings, including monocyclic or bicyclic groups such as phenyl, biphenyl or naphthyl. When containing two aromatic rings (bicyclic, etc.), the aromatic rings of the aryl group may be joined at a single point (e.g., biphenyl), or fused (e.g., naphthyl). The aryl group may be optionally substituted by one or more substituents, e.g., 1 to 5 substituents, at any point of attachment. The substituents may be, for example, C₁₋₆ alkyl, OH, -NH₂, C₁₋₆ alkoxy, -NH-(C₁₋₆ alkyl) or halogen.

The term halo-substituted phenyl group generally refers to fluorobenzene, chlorobenzene, bromobenzene, iodobenzene, difluoro benzene, dichlorobenzene, dibromo benzene or diiodo benzene groups, which are attached to the structure of the compounds of formula (A), formula (I) or formula (II) as indicated in these formulae.

### Heterocyclyl

The term heterocyclyl refers to a heterocyclic ring i.e. a ring consisting of carbon atoms and heteroatoms. In the context of the present invention, the terms "heterocycle" and "heterocyclic ring" are used interchangeably. In this context the term 'heteroatom' means O, S, N, Si or B (Boron). Optionally, the term 'heteroatom' means O, S or N. Preferably, the heterocyclic ring is a C₃₋₁₀ heterocyclyl. The term "C₃₋₁₀ heterocyclyl" as used herein, pertains to a heterocyclic compound, which has from 3 to 10 ring atoms, of which from 1 to 5 are ring heteroatoms. In certain embodiments, each ring has from 3 to 7 ring atoms, of which from 1 to 4 are ring heteroatoms. The ring may be saturated or unsaturated, and may be bridged or unbridged. The ring may be a fused ring or a single ring. For the avoidance of doubt, substituents on the heterocycloalkyl ring may be linked via either a carbon atom or a heteroatom. The heterocyclyl group may be optionally substituted by one or more substituents, e.g., 1 to 5 substituents, at any point of attachment, for example a halo-substituted phenyl group.

In this context, the prefixes (e.g. C3-10 C3-7, C5-6, etc.) denote the number of ring atoms, or range of number of ring atoms, whether carbon atoms or heteroatoms. For example, the term "C5-6 heterocyclyl", as used herein, pertains to a heterocyclyl group having 5 or 6 ring atoms.

Examples of monocyclic heterocyclyl groups include, but are not limited to, those derived from:
N1: aziridine (C3), azetidine (C4), pyrrolidine (tetrahydropyrrole) (C5), pyrroline (e.g., 3-pyrroline, 2,5-dihydropyrrole) (C5), 2H-pyrrole or 3H-pyrrole (isopyrrole, isoazole) (C5), piperidine (C6), dihydropyridine (C6), tetrahydropyridine (C6), azepine (C7);
O1: oxirane (C3), oxetane (C4), oxolane (tetrahydrofuran) (C5), oxole (dihydrofuran) (C5), oxane (tetrahydropyran) (C6), dihydropyran (C6), pyran (C6), oxepin (C7);
S1: thiirane (C3), thietane (C4), thiolane (tetrahydrothiophene) (C5), thiane (tetrahydrothiopyran) (C6), thiepane (C7);
O2: dioxolane (C5), dioxane (C6), and dioxepane (C7);
O3: trioxane (C6);
N2: imidazolidine (C5), pyrazolidine (diazolidine) (C5), imidazoline (C5), pyrazoline (dihydropyrazole) (C5), piperazine (C6);
N1O1: tetrahydrooxazole (C5), dihydrooxazole (C5), tetrahydroisoxazole (C5), dihydroisoxazole (C5), morpholine (C6), tetrahydrooxazine (C6), dihydrooxazine (C6), oxazine (C6);
N1S1: thiazoline (C5), thiazolidine (C5), thiomorpholine (C6);
N2O1: oxadiazine (C6);
O1S1: oxathiole (C5) and oxathiane (thioxane) (C6); and,
N1O1S1: oxathiazine (C6).

Preferably, the heterocyclic ring is a heteroaryl ring (i.e. a heteroaryl group).

Preferably, the heteroaryl ring is a 5-membered monocyclic heteroaryl ring, e.g. pyrrolyl; diazolyl, such as imidazolyl or pyrazolyl; triazolyl, such as 1 ,2,3-triazolyl or 1 ,2,4-triazolyl; or tetrazolyl. More preferably, the heteroaryl ring is a substituted or unsubstituted imidazolyl or a substituted or unsubstituted 1 ,2,3-triazolyl ring, and more preferably it is a substituted or unsubstituted 1 ,2,3-triazolyl ring.

Optionally, the heteroaryl ring is a 6-membered monocyclic heteroaryl ring, such as piridyl, diazinyl, triazinyl or tetrazinyl.

Preferably, the heteroaryl ring is a 9-membered bicyclic heteroaryl ring. Preferably, the 9-membered bicyclic heteroaryl ring is formed by a 6-membered cycle fused to a 5-membered cycle, such as a substituted or unsubstituted benzotriazole.

Optionally, the heteroaryl ring is a 10-membered bicyclic heteroaryl ring. Preferably, this 10-membered bicyclic heteroaryl ring is formed by a 6-membered cycle fused to a 6-membered cycle, and more preferably one of the 6-membered cycles comprises no nitrogen ring atoms.

### Alkoxy

The term "alkoxy" refers to an O-R group wherein R is an alkyl group. For example, a C₁₋₆ alkoxy pertains to an O-R group, wherein R is an C₁₋₆ alkyl group. Examples of C₁₋₆ alkoxy groups include, but are not limited to, OMe, OEt (ethoxy), -O(nPr) (n-propoxy), -O(iPr) (isopropoxy), O(nBu) (n-butoxy).

### Oxadiazole ring

The term "oxadiazole ring" may refer to 1,2,3-oxadiazole, 1,2,4-oxadiazole, 1,2,5-oxadiazole or 1,3,4-oxadiazole.

If not indicated otherwise "%" refers to weight-%.

### Compound of formula (A)

The present invention is focused on a compound of formula (A) for use in the treatment of Rett syndrome (RTT): 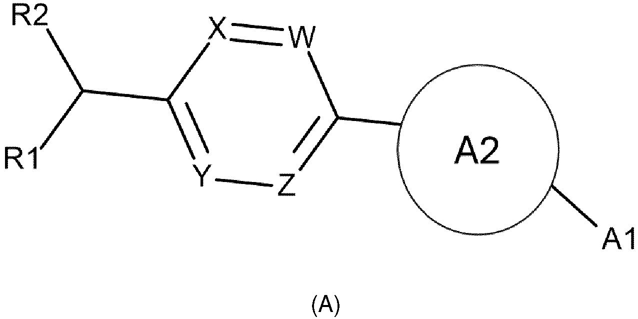 or a salt, solvate, stereoisomer or prodrug thereof,
wherein
A1 is CF₃, CHF_{2,} CH₂F
A2 is an oxadiazole ring;
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (A).

Methods of preparing a compound of formula (A) are described in e.g. WO/2020212479, the contents of which are incorporated herein by reference.

The skilled artisan understands that, when it is stated that W, X, Y or Z is CH, it is the carbon atom, and not the hydrogen atom, of said CH group that is one of the ring members of the six membered ring which comprises said W, X, Y or Z.

Preferably, the present invention is focused on a compound of formula (B) for use in the treatment of Rett syndrome (RTT): 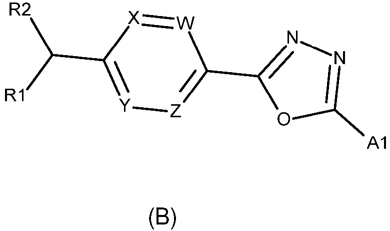 or a salt, solvate, stereoisomer or prodrug thereof,
wherein
A1 is CF₃, CHF_{2,} CH₂F
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (B);

More preferably, the present invention is focused on a compound of formula (I) for use in the treatment of Rett syndrome (RTT): 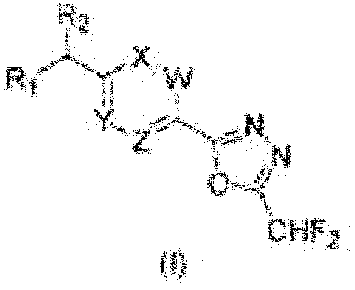 or a salt, solvate, stereoisomer or prodrug thereof, wherein
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (I).

In one aspect, two of W, X, Y or Z are N, and the remainder of W, X, Y and Z are each CH, in other words, the six membered ring which comprises said W, X, Y and Z is a diazine, and in particular it is a pyridazine, a pyrimidine or a pyrazine. A preferred diazine is a pyrimidine, and more preferably a pyrimidine wherein X and Y are N and Z and W are CH.

In a preferred aspect, one of W, X, Y or Z is N, and the remainder of W, X, Y and Z are each CH, in other words, the six membered ring which comprises said W, X, Y and Z is a pyridine. In a particular aspect, it is a pyridine wherein Z or W is N, and the remainder of W, X, Y and Z are each CH. In a more preferred particular aspect, it is a pyridine wherein X or Y is N, and the remainder of W, X, Y and Z are each CH.

In another preferred aspect, all of W, X, Y and Z are CH. R₁ is H; unsubstituted or substituted alkyl; or halogen

When R₁ is halogen, a preferred halogen is Cl or F. Preferably, the halogen is Cl. Alternatively, the halogen is F.

I

Preferably, R₁ is H.

Any of the above options for R₁ may be combined with any of the above W, X, Y or Z embodiments.

Particularly preferably, the six membered ring which comprises W, X, Y and Z is a pyridine wherein X or Y is N, and the remainder of W, X, Y and Z are each CH; and R₁ is H.

Optionally, all of W, X, Y and Z are CH; and R₁ is H.

Optionally, R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (I).

Preferably, the heterocyclic ring is a heteroaryl ring.

Preferably, the heteroaryl ring is a 5-membered monocyclic heteroaryl ring, e.g. pyrrolyl; diazolyl, such as imidazolyl or pyrazolyl; triazolyl, such as 1,2,3-triazolyl or 1,2,4-triazolyl; or tetrazolyl. More preferably, the heteroaryl ring is an imidazolyl or a 1,2,3-triazolyl ring, and most preferably it is a 1,2,3-triazolyl ring.

Preferably, the six membered ring which comprises W, X, Y and Z is a pyridine wherein X or Y is N, and the remainder of W, X, Y and Z are each CH; R₁ is H; and R₂ is 1,2,3-triazolyl; preferably R₂ is a substituted 1,2,3-triazolyl.

Optionally, R2 is a 9-membered bicyclic heteroaryl ring. Preferably, the 9-membered bicyclic heteroaryl ring is formed by a 6-membered cycle fused to a 5-membered cycle. More preferably, it is a nitrogen atom of the 5-membered cycle that forms the bond to the rest of formula (I).

Preferably, in the fused cycles, the fusion bond comprises no nitrogen atom, i.e. it is a carbon-carbon bond. This means that, when it is a nitrogen atom of the 5-membered cycle that forms the bond to the rest of formula (I), the 6-membered cycle can comprise from none to three nitrogen atoms (in its cycle structure, i.e. not including any nitrogen atoms comprised by any substituent of the cycle).

Preferably, the 6-membered cycle comprises no nitrogen atoms (including the fusion bond) and therefore the from 1 to 4 nitrogen ring atoms of R₂ must be from 1 to 3 nitrogen ring atoms and must all be comprised in the 5-membered cycle, (excluding the fusion bond). Examples of such heteroaryl rings are the indolyl, benzoimidazolyl, indazolyl and benzotriazolyl rings. More preferably, the heteroaryl ring is a benzoimidazolyl or benzotriazolyl ring. Most preferably, the heteroaryl ring is a benzotriazolyl ring.

In any of the aspects set out above, the 5-membered cycle may be an imidazolyl or a 1,2,3-triazolyl ring, and it is more preferably a 1,2,3-triazolyl ring.

Preferably, the six membered ring which comprises W, X, Y and Z is a pyridine wherein X or Y is N, and the remainder of W, X, Y and Z are each CH; R₁ is H; and R₂ is a benzimidazolyl or benzotriazolyl ring.

In any of the aspects outlined above, the R₂ heterocyclic ring may be unsubstituted, or it may be substituted at one or more available positions with a substituent or, where available, with more than one substituent.

Suitable R₂ substituent groups include:
- alkyl, alkoxy, thioalkoxy, and halogenated derivatives thereof;
- halogen;
- phenyl and phenyl substituted with alkyl, alkoxy, thioalkoxy or halogenated derivatives thereof,
   - -O, - C(=O)Ra or -C(=O)ORa, wherein Ra is an alkyl group as defined above, or a halogenated derivative thereof; and
   - pyridyl and pyridyl substituted with alkyl, alkoxy, thioalkoxy or halogenated derivatives thereof,
   - thiophenyl, furan or pyrrole and thiophenyl, furan or pyrrole substituted with alkyl, alkoxy, thioalkoxy or halogenated derivatives thereof.

The R₂ substituent is preferably a relatively apolar group. Therefore, optionally, the R₂ substituent group is selected from:
- alkyl, alkoxy, thioalkoxy, and halogenated derivatives thereof;
- halogen; and
- phenyl and phenyl substituted with alkyl, alkoxy, thioalkoxy or halogenated derivatives thereof,

Preferably, the compound of formula (A) is according to formula (II): 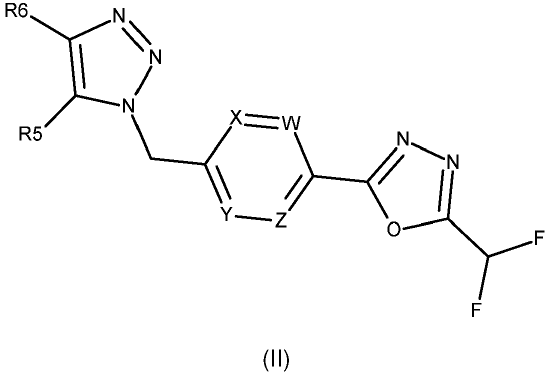 wherein
one or two of W, X, Y or Z is N and the remainder of W, X, Y and Z are each CH, or each W, X, Y and Z is CH;
and wherein
R5 is hydrogen or a substituted or unsubstituted C₁₋₆ alkyl,
R6 is a substituted or unsubstituted 5 to 8 membered aromatic group or a substituted or unsubstituted 5 to 8 membered heteroaryl group;
or R5 and R6 together with the carbons to which they are attached form a substituted or unsubstituted 5 to 8 membered aromatic group
or a salt, solvate, stereoisomer or prodrug thereof.

The definitions set out above for substituted or unsubstituted C₁₋₆ alkyl and substituted or unsubstituted 5 to 8 membered aromatic groups also apply to the compound of formula (II).

More preferably, the compound of formula (A) is according to formula (II), wherein one of W, X, Y or Z is N and the remainder of W, X, Y and Z are each CH and wherein
R5 is hydrogen and R6 is a halo substituted phenyl; or
R5 and R6 together with the carbons to which they are attached form a halo substituted phenyl.

Preferably, the compound of formula (A) is according to formula (II), wherein X or Y is N and the remainder of W, X, Y and Z are each CH;
and wherein
R5 is hydrogen and R6 is a halo substituted phenyl; or
R5 and R6 together with the carbons to which they are attached form a halo substituted phenyl.

Preferably, the compound of formula (II) is according to formula (III) 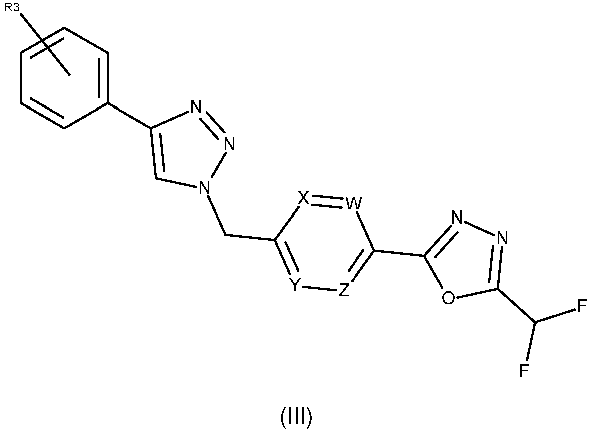 wherein
one or two of W, X, Y or Z is N and the remainder of W, X, Y and Z are each CH, or each W, X, Y and Z is CH;
and wherein R³ is absent, halo, C₁₋₆ alkyl, C₁₋₆ haloalkyl, OH, NH₂
or a salt, solvate, stereoisomer or prodrug thereof.

Preferably, the compound (HDAC6 inhibitor) has a formula according to formula (IV) 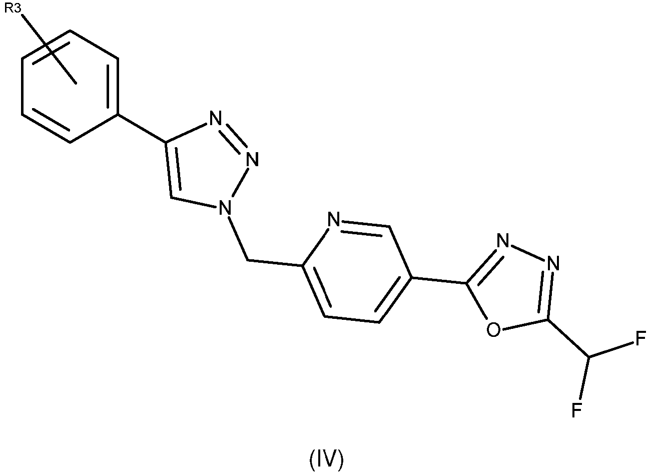
wherein R3 is halo
or a salt, solvate, stereoisomer or prodrug thereof.

Most preferably the compound (HDAC6 inhibitor) is 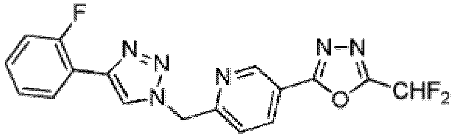 or a salt, solvate, stereoisomer or prodrug thereof

Alternatively, the compound (HDAC6 inhibitor) is 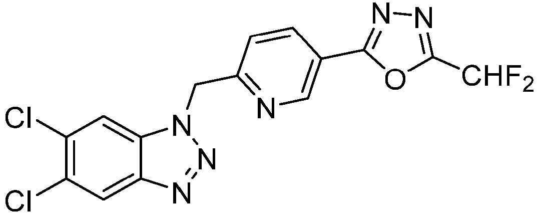 or a salt, solvate, stereoisomer or prodrug thereof

### Medical Use, Methods of Treatment

In a further aspect, the present invention relates to a compound of formula (A) 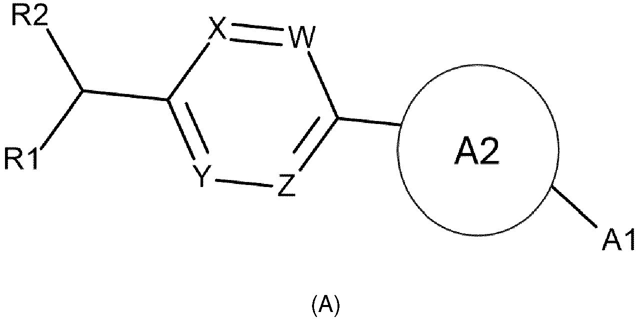 or a salt, solvate, stereoisomer or prodrug thereof,
wherein
A1 is CF₃, CHF_{2,} CH₂F
A2 is an oxadiazole ring;
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (A); for use in the manufacture of a medicament for the treatment of Rett syndrome (RTT).

The specific and preferred aspects of the compound of formula (A) for use in the treatment of RTT described above also apply for the compound of formula (A) for use in the manufacture of a medicament for the treatment of RTT.

In a further aspect, the present invention relates to a method of treatment of Rett syndrome (RTT) comprising administering a compound of formula (A): 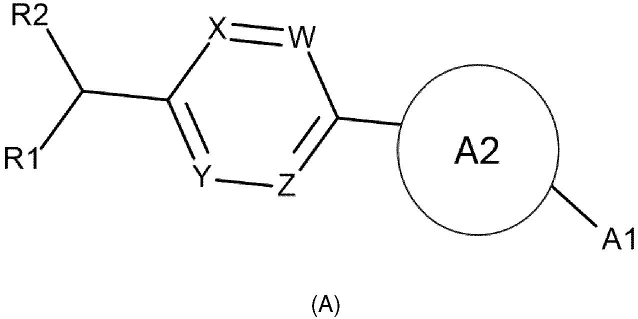 or a salt, solvate, stereoisomer or prodrug thereof,
wherein
A1 is CF₃, CHF_{2,} CH₂F
A2 is an oxadiazole ring;
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted, aromatic or non-aromatic heterocyclic ring, wherein the ring comprises from 1 to 4 nitrogen atoms, and wherein it is one of these ring nitrogen atoms of the R₂ group that forms the bond to the rest of formula (A) to a subject in need of such treatment.

The specific and preferred aspects of the compound of formula (A) for use in the treatment of RTT described above also apply for the compound of formula (A) administered to a subject in the method described above.

### Administration

The method of administration of the compound of formula (A) (HDAC6 inhibitor) is not particularly limited.

Optionally, the compound of formula (A) is administered orally, by injection (e.g. intravenously, intramuscularly, subcutaneously), sublingually, rectally, vaginally, nasally or by inhalation.

Preferably, the compound of formula (A) is administered orally, nasally or by inhalation.

Without being bound by any theory, it is believed that oral, nasal or administration by inhalation are advantageous over other routes of administration (e.g. intravenous, intramuscular or subcutaneous) as they are less invasive and easier for the patent.

Most preferably, the compound of formula (A) is administered orally. Without being bound by any theory, it is believed that oral administration is generally the most convenient for patients and is facilitated by the oral availability of HDAC6 inhibitors, such as the compound of formula (V).

### Subject/Dosing

The subject for administration may be any animal. Preferably, the subject is a mammal, such as a rat, mouse, feline, canine, equine, porcine, ovine, bovine, primate or human. Most preferably, the subject is a human patient.

In general, the compounds (HDAC6 inhibitors) according to the present invention are well tolerated in mammalian models.

In general, the effective amount of the compound of formula (A) to be administered will depended on a range of factors, such as the severity of the disorder being treated and the subject's weight. The active compounds will normally be administered one or more times a day for example 1, 2, 3, or 4 times daily, with typical total daily doses in the range from 0.01 up to 1000 mg/kg/day.

Preferably, the compound of formula (A) is administered to human patients at a dosage of 0.5 mg/kg, or at least 1 mg/kg, or at least 2 mg/kg, or at least 10 mg/kg, such as about 20 mg/kg. Preferably, from 0.5 mg/kg to 30 mg/kg, more preferably from 0.5 mg/kg to 20 mg/kg, most preferably from 1 mg/kg to 20 mg/kg.

Preferably, the compound of formula (A) is administered to human patients at a dosage of from 50 to 2000 mg, preferably from 200 to 1000 mg, more preferably from 500 to 1000 mg, more preferably from 700 to 950 mg per day, such as around 900 mg per day.

Each of the compounds (HDAC6 inhibitors) according to formula (A) has a minimum toxic concentration (in mg/kg), which may be determined using routine toxicology testing on a rodent model and extrapolated to give a minimum toxic concentration (in mg/kg) for a 70 kg human subject. Each of the compounds (HDAC6 inhibitors) according to formula (A) also have a minimum effective concentration (in mg/kg) which is the minimum concentration required to effectively suppress the symptoms of RTT determined using routine testing in a rodent model and extrapolated to give a minimum effective concentration (in mg/kg) for a 70 kg human subject. Taking these two figures together, it is possible to generate an Effectivity range, according to formula (C): $Effectivity range \left(E\right) = \frac{Minimum concentration required to effectively supress the symptoms of RTT}{Minimum toxic concentration}$

Preferably, the effectivity range is at least 3, preferably at least 5, more preferably at least 7, more preferably at least 10, most preferably at least 20.

### Pharmaceutical compositions

The compound (HDAC6 inhibitor) according to formula (A) according to the present invention is preferably administered as part of a pharmaceutical composition. The said pharmaceutical composition may comprise a compound of formula (A) and one of more other pharmaceutically acceptable ingredients.

Pharmaceutically acceptable ingredients, may include, but are not limited to: pharmaceutically acceptable carriers, diluents, excipients, adjuvants, buffers, pH modifiers, preservatives, antioxidants, bacteriostats, stabilisers, suspending agents, solubilisers, surfactants (e.g., wetting agents), colouring agents, and isotonicising solutes (i.e., which render the formulation isotonic with the blood, or other relevant bodily fluid, of the intended recipient). Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 18th edition, Mack Publishing Company, Easton, Pa., 1990; and Handbook of Pharmaceutical Excipients, 2nd edition, 1994.

Optionally, the pharmaceutical composition comprises a compound of formula (A) and an excipient. The excipient may be selected from the group comprising stabilizers, surfactants, buffering agents, antimicrobial preservatives, protectants, antioxidants, chelating agents, bulking agents and mixtures thereof.

The pharmaceutical composition may also comprise counter-ions and salts, such as sodium counter ions, chloride ions or NaCl dissolved is solution.

In one aspect of the invention the compound (HDAC6 inhibitor) according to formula (A) for use in treating RTT may be present as a tablet (such as an orally disintegrating table), dragee, capsules, lozenge, syrup, solution, film, powder or paste.

Preferably, the compound (HDAC6 inhibitor) according to formula (A) is present as a tablet.

Alternatively, the compound (HDAC6 inhibitor) according to formula (A) for use in treating RTT may be present as a liquid when used for injection.

The compound (HDAC6 inhibitor) may also be present as part of a kit comprising the compound (HDAC6 inhibitor) and a method of administrating the compound to a patient (such as a kit comprising a liquid form of the compound and a syringe to administer the liquid to a patient).

### Second pharmaceutical agents

Optionally, the compound of formula (A) or a salt, solvate, stereoisomer or prodrug thereof is administered together with a one or more other active agents, for example, one or more other therapeutic or prophylactic agents. In some aspects, these agents may be utilized to provide a combination therapy.

The compound of formula (A) may be administered to a subject simultaneously, sequentially or separately with one or more other active agents.

In particular, the compound of formula (A) may be administered with a second pharmaceutical agent. Preferably, the second pharmaceutical agent is a neuropeptide analogue.

Examples of neuropeptide analogues are Trofinetide, Neurokinin A, Desmopressin, Carbetocin, Des-octanoyl ghrelin.

Preferably, the neuropeptide analogue is Trofinetide.

When the compound of formula (A) is administered as part of a composition, the second pharmaceutical agent may be part of the same composition or may be provided as a separate composition and can be administered at the same time or at different times. Preferably, the second pharmaceutical agent is part of the same composition as the compound of formula (A).

For example, disclosed herein is a pharmaceutical composition comprising a first pharmaceutical agent comprising a compound of formula (A) a salt, solvate, stereoisomer or prodrug thereof; a second pharmaceutical agent which is a neuropeptide analogue; and a pharmaceutically acceptable carrier, excipient or diluent. In some aspects, the second pharmaceutical agent is Trofinetide. These pharmaceutical compositions are intended for use in the treatment of RTT, or in the manufacture of a medicament for the treatment of RTT. Alternatively, the present invention relates to a method of treatment of RTT comprising administering the above pharmaceutical compositions to a subject in need of such treatment.

Exemplary molar ratios of the first pharmaceutical agent (i.e., a compound of formula (A)) to the second pharmaceutical agent are from 1:30 to 30:1, preferably from 1:10 to 10:1, preferably from 1:5 to 5:1, more preferably from 1:2 to 2:1. In some aspects of the disclosure, the molar ratio of the first pharmaceutical agent to the second pharmaceutical agent is about 1:1.

### Indications

The present application relates to the use of a compound (HDAC6 inhibitor) according to formula (A) in the treatment of RTT or in the manufacture of a medicament for the treatment of RTT. Alternatively, the present application relates to a method of treatment of RTT comprising administering a compound (HDAC6 inhibitor) according to formula (A) to a patient in need of such treatment.

As set out above RTT may be characterised by symptoms including loss of speech and motor skills, difficulty walking, seizures, gait abnormalities, repetitive hand movements and irregular breathing patterns.

The compounds (HDAC6 inhibitors) according to formula (A), may be useful in the treatment of a range of symptoms associated with RTT. Therefore, in a certain aspect the present invention relates to a compound according to formula (A) for use in the treatment of RTT wherein the compound is effective at treating a decrease in mobility associated with RTT. In a certain aspect, the present invention relates to a compound according to formula (A) for use in the treatment of RTT wherein the compound is effective at treating tremor associated with RTT. In a certain aspect, the present invention relates to a compound according to formula (A) for use in the treatment of RTT wherein the compound is effective at treating walking problems (gait issues) associated with RTT. In a certain aspect, the present invention relates to a compound according to formula (A) for use in the treatment of RTT wherein the compound is effective at treating breathing difficulties associated with RTT. In a certain aspect, the present invention relates to a compound according to formula (A) for use in the treatment of RTT wherein the compound is effective at treating limb clasping associated with RTT.

Preferably, the compound according to formula (A) for use in the treatment of RTT is effective in treating tremor associated with RTT or preventing the development of tremor associated with RTT.

Preferably, the compound according to formula (A) for use in the treatment of RTT is effective in treating mobility difficulties associated with RTT or in improving mobility in a subject suffering from RTT.

The compounds according to formula (A) for use in the treatment of RTT may also be effective in preventing loss of neuronal branch points associated with RTT.

The above specific indications also apply to compounds according to formula (A) for use in the manufacture of a medicament for the treatment of RTT and to methods of treatment of RTT involving administering a compound according to formula (A).

### Preferred embodiments

Particularly preferred embodiments include:

A compound (HDAC6 inhibitor) for use in the treatment of RTT, wherein the compound has a formula according to formula (III) 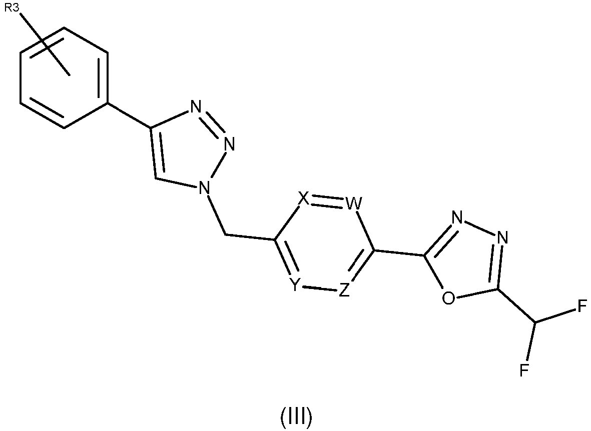 wherein
one or two of W, X, Y or Z is N and the remainder of W, X, Y and Z are each CH, or each W, X, Y and Z is CH;
and wherein R³ is absent, halo, C₁₋₆ alkyl, C₁₋₆ haloalkyl, OH, NH₂.

Particularly, preferred is compound (HDAC6 inhibitor) for use in the treatment of RTT, wherein compound is:

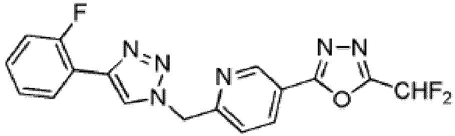

Preferably, wherein the compound (HDAC6 inhibitor) is administered orally to a subject.

Preferably, wherein the compound (HDAC6 inhibitor) is administered orally as a tablet.

Preferably, wherein the compound (HDAC6 inhibitor) for use in the treatment of RTT is effective in treating tremor associated with RTT or preventing the development of tremor associated with RTT.

As will be appreciated by one of skill in the art, features and preferred embodiments of one aspect of the invention will also pertain to other aspects of the invention.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilized for realizing the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value.

### EXAMPLES

In order to better understand the nature of this invention, a number of illustrative examples will now be described.

The scope of the invention is not limited to the examples provided below. The examples merely demonstrate the effectiveness of the invention.

### Examples

### Materials and methods

### Cellular models

For all assays, the neural progenitor ReNcell VM cell line (SCC008, Merck Millipore) (derived from the ventral mesencephalon of a 10-week gestational human male fetal brain immortalized with *v-myc*) was used in either its parental version (wild-type, WT), or in its CRISPR/Cas9 engineered versions that mutate the *MECP2* gene (the full knock-out *MECP2*-KO or the point mutant *MECP2*-R133C¹). When HDAC6i were used in the mutant cells, WT cells were always mock-treated in parallel as reference.

### Cell culture

Both wild-type and mutant ReNcell cultures were maintained as progenitors cells in complete media: DMEM-F12 (L0093-500, Biowest), B27 complete vitamins (17504-044, Invitrogen), antibiotic/antimycotic (L0010-100, Biowest) and 0.1% heparin (Stem Cell Technologies) with EGF (20 ng/ml, SRP3027) and bFGF-2 (20 ng/ml, SRP4037) (Sigma-Aldrich), at 37ºC in a humidified 5% CO₂ atmosphere. Before plating, culture dishes were coated with laminin (10 µg/ml in PBS, Sigma Aldrich) by incubating the plate surface overnight at 37ºC. The medium was replaced every other day, and the culture was passed every 3-6 days when 90% confluence was reached. For the induction of spontaneous (free) differentiation, the cells were briefly washed with DMEM/F-12 and given incomplete media (without growth factors EGF and bFGF). To force glutamatergic differentiation, cells containing the plasmids TetO-hNGN2-P2A-eGFP-T2A-PuroR and CMV-rtTA (as described in Siqueira, E. et al. Mol. Ther. - Nucleic Acids 27, 621-644 (2021)) were seeded on laminin-coated plates. 24 h after seeding, the drugs or vehicle (at the indicated concentrations) and doxycycline (1 µg/ml) were administrated to the cells, followed by Puromycin (0.5 µg/ml) the next day. After 24 h of Puromycin selection, EGF and FGF-2 were withdrawn from the media, and cells were allowed to differentiate for the indicated days. Half the medium was changed every day maintaining doxycycline and drugs or vehicle concentration.

### Drug treatment

Treatment with the drugs was conducted at the indicated concentrations and time-points; an equivalent concentration of dimethyl sulfoxide (DMSO) vehicle served as control. The drugs used were: QTX153 (QCI012-405, Quimatryx), ACY-1215 (205808, MedKoo Biosciences), ACY-775 (145340, TargetMol), Tubastatin A (148012, TargetMol), Trofinetide (149993, TargetMol).

### Viability assays

Cell metabolic activity was assessed using the MTT (3-(4,5-dimethyl-2-thiacetaolyl)-2,5-biphenyl-2H-tetrazolium bromide) assay. Cells were seeded into 96-well plates (2000 cells per well), and 24 h later, the drugs were added. After 72 h, 10 µl of 5 mg/ml MTT was added to each well containing 150 µl of medium. After incubation for 3 h at 37°C, 100 µl of lysis buffer (20% sodium dodecyl sulfate (SDS), 50% N, N-dimethylformamide, pH 4.7) was added, and the mixture was further incubated overnight. Absorbance was measured at 560nm (MTT) and 620nm (for background reference).

### SDS-PAGE and Western blot

Cell pellets were resuspended in Laemmli buffer (2% SDS, 10% glycerol, 0.01% bromophenol blue and 60 mM Tris-HCl pH 6.8), sonicated, boiled and run on an SDS gel (10% separating, 5% stacking).

Proteins were transferred to a 0.2 µm nitrocellulose membrane (10600001, GE Healthcare) and incubated overnight at 4°C with primary antibody diluted in 5% skimmed milk in PBS containing 0.1% Tween^{®} 20 (663684B, Atlas Chemical Inc.). After three washes with PBS containing 0.1% Tween^{®} 20, membranes were incubated for 1 h at RT (room temperature) in a bench-top shaker with the secondary antibodies conjugated to horseradish peroxidase anti-rabbit IgG (1:10000, A0545; Sigma-Aldrich) or anti-mouse IgG (1:10000, NA9310, GE HealthCare), or IRDye^{®} 680/800 anti-rabbit (926-32213, LI-COR) or anti-mouse (926-68070, LI-COR). ECL reagents (Luminata-HRT; Merck Millipore) were used to visualize the proteins. Primary antibodies used were: acetyl-α-tubulin (1:2000, T6793, Sigma-Aldrich), α-tubulin (1:1000, ab52866, Abcam), Synapsin-1 (1:1000, 5297S, Cell Signalling), PSD95 (1:1000, 3450S, Cell Signalling), Synaptophysin (1:1000, 5461S, Cell Signalling), VGlut1 (1:1000, 135 302, Synaptic Systems), β-ACTIN HRP (1:15000, a3854, Sigma-Aldrich).

### Neurosphere formation assay

Progenitor cells were cultured with complete medium (with EGF and FGF-2) in non-coated flasks to stimulate the formation of spheres. Spontaneous sphere formation occurs after 24h of plating, and cells were allowed to expand for 7 days before imaging. Images (n=16 per condition) were acquired with a DM IL LED Fluorescence microscope (Leica), and the spheres' diameter was measured using the ImageJ software (v1.53t).

### Analysis of neuronal morphology

Neuronal morphology was revealed by immunofluorescence (IF) staining with an anti-MAP2 antibody as follows. 10,000 cells were seeded on laminin-coated coverslips placed in 12-well plates. Cells under different treatment conditions were differentiated for 7 days following the glutamatergic protocol. At the day of the experiment (day 7 of differentiation), cells were fixed using 4% paraformaldehyde (PFA) added to the medium for 20 min at RT. Each sample was blocked with 5% BSA and 5% goat serum in 0.03% Triton X-100 in modified PBS (PBS with 1mM CaCl₂ and 1mM MgCl₂) for 1 h at RT. After that, the primary antibody (MAP2 1:400, #8707, Cell Signaling) incubation was carried out overnight (12-16 hours) at 4ºC diluted in the blocking solution. The next morning, secondary antibody (1:1000 anti-rabbit Alexa Fluor 647, ab150075, Abcam) was incubated for 1 h 30 min at RT in the dark. Samples were always washed three times between steps using modified PBS for 5 min each at RT. DAPI was used for nuclei staining with a 5 min incubation (1:10,000) and two 5 min washes in MiliQ water were performed. After that, coverslips were mounted on glass slides using Anti-Fade mounting medium and left overnight at RT in the dark to dry.

The images were acquired in Leica Stellaris 8 microscope and were analyzed in ImageJ software (v1.53k). Neurons were reconstructed in NeuroStudio Software (version 0.9.92): A three-dimensional dendritic network was built with a Sholl analysis by automated segmentation, where concentric circles are drawn from soma (over a length of 20 µm with concentric circles of 10 µm) throughout the projection's length. Neuronal components are categorized (soma, neurites and branching points) and represented by spheres. An estimation of spines density was retrieved (approximately n=15 neurons per condition and detailed in the Figure Legend of each experiment).

### Statistical analysis

Drug treatment experiments were performed at least in triplicates. Graphs and statistical analyses were obtained with Graphpad Prism 8.2.0. In all graphs, results are represented as the mean ± SD or ± SEM, as indicated, and comparative analyses of differences between experimental groups were performed using Mann-Whitney U test or unpaired samples t-tests and one-way ANOVA with a Tukey or Bonferroni post hoc test for intergroup comparisons. Results were considered significant for values of p < 0.05 (*), < 0.01 (**), < 0.001 (***), or < 0.0001 (****).

### Mouse colony

Experiments were performed on the B6.129P2(c)-MeCP2tm1+1 Bird mouse model for RTT (Guy, J et al. Nat. Genet. (2001) doi:10.1038/85899). The mice were purchased from Jackson Laboratories (stock number 3890) and maintained on a C57BL/6J background. Mice were kept under specific pathogen-free conditions in accordance with the recommendations of the Federation of European Laboratory Animal Science Associations. Lighting conditions (lights on from 08:00-20:00 h) and temperature (22°C) were kept constant. Animals were allowed ad *libitum* access to food and water and were inspected every day. All procedures and experiments were approved by the Ethics Committee for Animal Experiments of the IGTP Centre (project #12155), under the guidelines of Spanish animal welfare laws. Mice were euthanized in accordance with the Guidelines for Humane Endpoints for Animals Used in Biomedical Research. Tissue samples for analyses were obtained from treated hemizygous *Mecp2*-null males (*Mecp2*^{*-*/}*^{y},* KO) and their wild-type (WT) littermates at end-point (at about 7-8 week of age). Tissues were frozen on dry ice immediately after dissection and stored at -80 °C until use.

### Drug administration

*MECP2*-KO males of similar litters were randomly distributed into the control (vehicle-treated, vehicle = NMP:Solutol HS-15:normal saline (5:5:90)) and drug-treated animals (QTX153 in vehicle) until reaching the desired n. Animals were administered from 4 weeks of age until end point, from Monday to Friday, with a single daily dose of 160 mg/kg drug substance. Animals were administered from 10 weeks of age until end point, and received the drug on a Monday, Wednesday, Friday schedule, with a single daily dose of 160 mg/Kg. The method used was oral administration by forced ingestion: briefly, a 30 mm long, Gauge 20 (0.9 mm) diameter metal gastric tube with a rounded catheter was employed. The tube was attached to a syringe and handled like a needle in an injection. Once the tube and syringe were loaded with the appropriate volume of solution (the maximum volume to be administered is 0.3-0.4ml), the animal was immobilized by grasping the skin on the back with the thumb and index finger so that the head, neck, and thorax form a straight line. Next, the tube was gently inserted through the mouth, navigating towards the oesophagus and stomach until three-quarters of the tube had been inserted, and then the syringe contents were dispensed as quickly as possible and the tube was gently withdrawn. Mice were weighted daily as part of their wellbeing control, and for dose calculation. End-point was determined by the general deterioration of the animal or the need to take samples for analysis.

### Phenotypic scoring

Mice under treatment were monitored twice a week for symptoms associated with the RTT phenotype. The scoring method, based on Guy et al Nat. Genet. (2001) doi:10.1038/85899, focuses on mobility, gait, hindlimb clasping, tremor, breathing and general conditions. Each of the six symptoms were scored from 0 to 2 in male mice, with 0 indicating the symptom was absent and 2 indicating it was severe. The end-point was reached when the animal reaches the highest score for any of the last three criteria (tremor, breathing, and general condition). Additionally, if the animal loses 20% of its body weight during the experiment, it was also sacrificed. Score tests were always performed at the same time and before treatment and were blind to treatment status.

### Sample preparation for Western Blot

For mouse brain tissue samples, protein extraction was performed using the CelLytic^{™} MT Buffer (C3228, Sigma Aldrich) with Complete Protease Inhibitor Tablets (04693116001, Roche). Samples were sonicated, incubated for 2 hours at 4ºC on a shaker, and run on an SDS gel (10% separating, 5% stacking). Western blotting was performed as described above for cultured cells.

### Immunofluorescence

Mouse brain samples were frozen with OCT and then sectioned at 25-30 µm using a cryostat. The sections were placed on slides and fixed by immersing them in 4% PFA diluted with PBS for 30 minutes at RT. Three 10-minute washes with PBS were performed. Blocking was done for 1 hour at RT with a solution of PBS + 20% FCS + 0.25% Triton X-100. The primary antibody was incubated for 48 hours at 4ºC in a solution of PBS + 2% FCS + 0.25% Triton X-100. Three 10-minute washes with PBS were performed. The secondary antibody (1:10,000 anti-rabbit Alexa Fluor 647, ab150075, Abcam) was incubated for 1 hour at RT, followed by three 10-minute washes with PBS. DAPI was used for nuclei staining with a 5-minute incubation (1:10,000), followed by three 10-minute washes in Milli-Q water. Mounting was performed on the glass slides using Anti-Fade mounting medium and left overnight at RT in the dark to dry. Primary antibodies used were: VGlut1 (1:500, 47181S, Cell Signaling), Psd95 (1:400, 2507S, Cell Signaling), BDNF (1:500, ab108319, Abcam). The images were acquired using a Leica Stellaris 8 microscope and analyzed with ImageJ software (v1.53k).

### Golgi-Cox staining method and analysis of neuronal morphology

Golgi-Cox impregnation was performed according to the manufacturer's instructions for the superGolgi kit (Bioenno Tech, LLC, ref: 003010). Mice that exhibited experimental endpoint symptoms were sacrificed by rapid cervical dislocation. Wild-type mice of similar age were also included as controls. The brains were quickly but carefully dissected, washed with ddH2O to remove blood from the surface, and the whole brain was immersed in a 10-fold volume of Golgi-Cox solution at room temperature in the dark. The solution was changed after 24 hours, and the brains were left in Golgi-Cox for another 10 days. Once impregnation was complete, the brains were rinsed with ddH2O and transferred to B-prepared post-impregnation buffer (10-fold volume) and stored at room temperature in the dark. The solution was renewed after one day of immersion and continued for one more day at room temperature. The brains were then stored in post-impregnation buffer at 4°C for 5-10 days. The brain was divided longitudinally into two hemispheres, and the cerebellum was separated into a separate block. Tissues were embedded in TFM^{™} tissue freezing medium (Electron Microscopy Sciences, ref: 72592), sectioned in a cryostat into 80-µm slices, and mounted on SuperFrost Plus^{™} adhesion slides (Epredia^{™}, ref: J1800AMNZ). After drying overnight, slices were stained according to the manufacturer's instructions for the superGolgi Kit. Sections were dehydrated with 100% EtOH and xylenes, a coverslip was then placed over the specimen on the microscope slide, to hold the specimen in place and protect it from contamination from the environment using Eukitt^{®} (Quick-hardening mounting medium, Sigma, ref: 03989) to attach the coverslip. An AxioScan7 scanner was used to acquire binary grayscale images of the tissue sections. The images were acquired with a 40x objective and 12 Z-stacks of 1.5 µm thickness each and were analyzed with ImageJ software (v1.53k). Fully impregnated neurons (n ~ 15 neurons per condition) were reconstructed using NeuroStudio software (version 0.9.92). The specific settings for the calculation of neurites (branching) were as follows: Attach Ratio: 1.300, Discretization Ratio: 1.0, and Min Length: 1.000. For the spines, the default load settings were used.

**Table 1: Structure of compounds tested within this application**

| Compound Name | Inventive/Comparative | Structure |
|---|---|---|
| QTX 153 | Inventive | 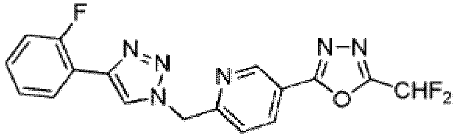 |
| QTX166 | Inventive | 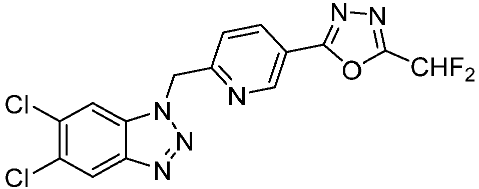 |
| QTX146 | Comparative | 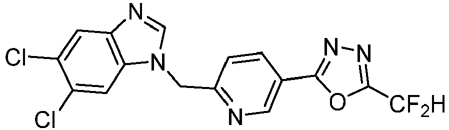 |
| ACY755 | Comparative | 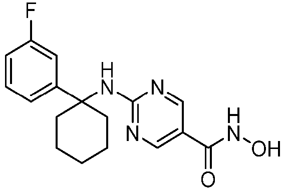 |
| ACY1215 | Comparative | 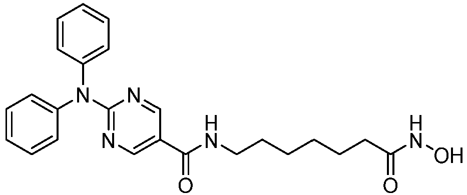 |
| Tubastatin A | Comparative | 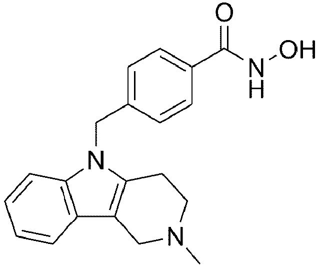 |
| Trofinetide | Comparative | 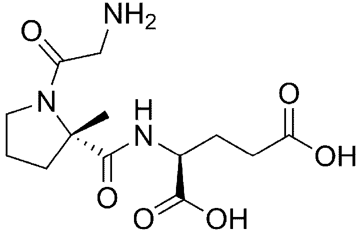 |

### EXAMPLE 1: In vitro toxicity assays in MECP2-KO RenCell cultures

To determine the potential toxicity of each drug in neural progenitor cultures, a titration of the concentration of each compound was performed and cell viability measured to determine the IC₅₀ of each drug. Briefyl: *MECP2*-KO ReN cells were cultured with each inhibitor for 72 hours. The concentration range used for all compounds was between 10⁻⁶ and 10² µM. For each concentration, 6000 cells were plated per well in a 96-well plate, with 8 wells analyzed per concentration.

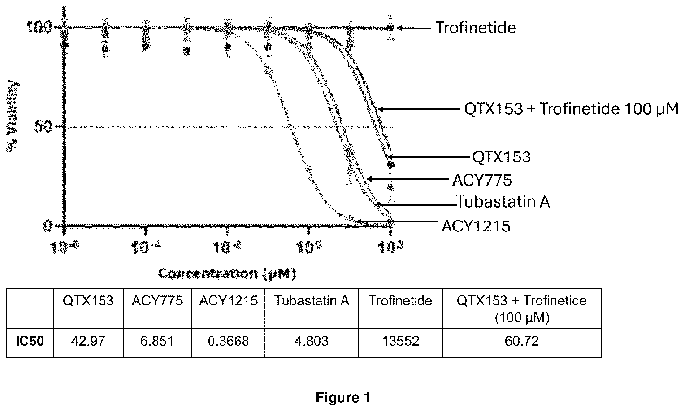

The results are summarized in the composite graph shown in Figure 1. IC₅₀ values are in µM. As shown, ACY1215 exhibited some toxicity with an IC₅₀ of 0.4 µM, whereas QTX153 displayed marginal toxicity only at very high concentrations (IC₅₀ ~ 50 µM). Trofinetide caused virtually no toxicity in ReNcells.

To test potential synergistic activity, the combination of QTX153 and Trofinetide was also tested, maintaining Trofinetide at 100 µM. The combination did not worsen the viability of the cells relative to either treatment alone. Based on these viability results, the working concentrations for each drug in subsequent experiments was established.

### EXAMPLE 2: Ability of HDAC6i to restore physiological acetylation levels of α-tubulin

The activity of QTX153, ACY775, ACY125, Tubustatin A and Trofinetide as inhibitors of HDAC6 function was tested using Western blot analysis, with acetylated α-tubulin levels (the canonical substrate of HDAC6) as the read-out. Acetylated α-tubulin levels in MECP2-KO cells were measured by Western blot after 48 hours of treatment with each compound. Mock-treated (DMSO-treated) cells are shown for comparison. Total α-tubulin was measured as a loading control, along with β-actin.

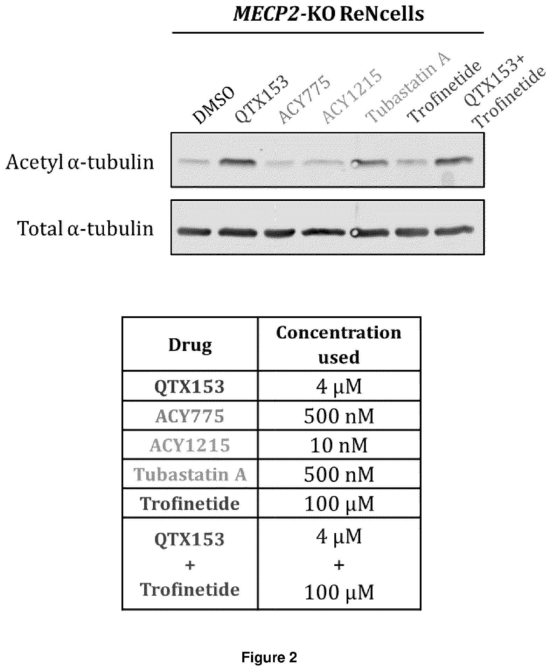

Initially, the maximum concentration that did not cause cell death for each drug was tested (Figure 2). The results indicated a marked increase in acetyl-tubulin levels after treatment with the maximum non-toxic doses of QTX153 and Tubastatin A. By comparison, non-toxic doses of ACY775 and ACY1215 could not restore α-tubulin acetylation. Trofinetide, not being an HDAC6 inhibitor, was not expected to alter tubulin acetylation levels. The combination of QTX153 and Trofinetide (both at high concentrations) restored acetylation to levels similar to QTX153 alone.

These results demonstrate that QTX153 can effectively restore acetylated α-tubulin levels, at a non-toxic dose.

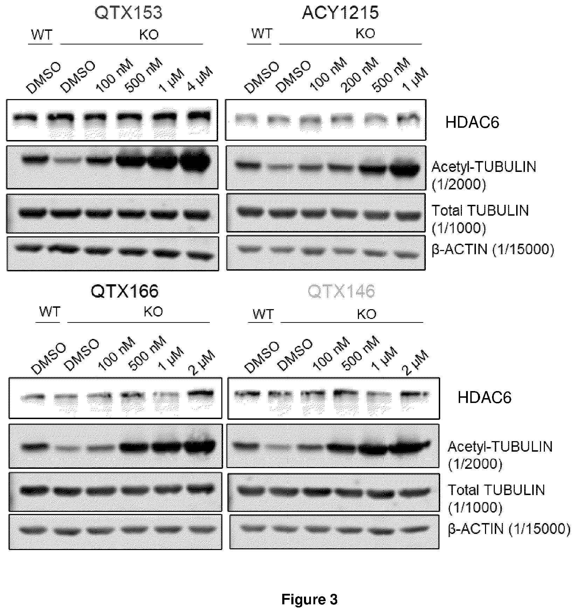

Next, a range of concentrations of QTX153 were tested to determine the lowest dose capable of restoring physiological levels of acetyl-α-tubulin, using wild-type neural progenitor ReNcells as a reference (see Figure 3). Briefly, dose response analysis of QTX153, ACY1215, QTX166 and QTX146 was conducted to assess ability to restore acetylated α-tubulin levels in MECP2-KO cells compared to wild-type (WT) cells. Acetylation levels were measured by Western blot after 48 hours of treatment with each compound, along with HDAC6 levels (which are not expected to change). Mock-treated (DMSO-treated) cells are shown for comparison. Total α-tubulin was measured as a loading control, along with β-actin.

As shown in Figure 3, doses of QTX153 as low as 100 nM rescued α-tubulin wildtype acetylation levels in MeCP2 cells with Rett syndrome (MECP2-KO cells). This dosage was much lower than the required dose of ACY1215. The other HDAC6 compounds of Quimatryx, QTX166 and QTX146, were able to rescue the α-tubulin acetylation levels at similar concentrations to ACY1215, with the cytotoxicity of QTX166 and QTX146 being much lower than that of ACY1215.

In view of these results, QTX153 was utilized at 200 nM in subsequent experiments aimed at determining its ability to restore α-tubulin acetylation during neural differentiation and in the presence of the MeCP2 point mutation R133C, which is one of the mutations present in Rett syndrome patients.

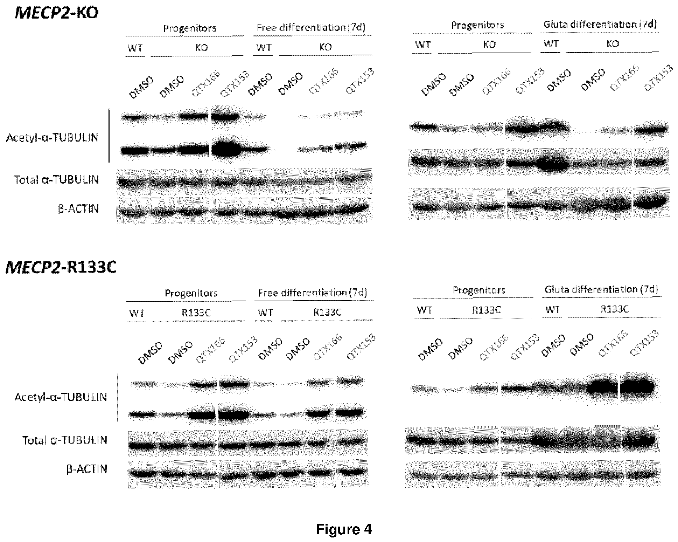

Subsequently, a 7-day spontaneous (free) differentiation protocol was applied, as well as a targeted glutamatergic neuronal protocol to assess the activity of each compound in a setting that mimics the early stages of brain development (Figure 4).

Briefly: acetylation levels were measured by Western blot after 48 hours of treatment with HDAC6 inhibitors QTX166 (400 nM) and QTX153 (200 nM). Blots for *MECP2*-KO cells are shown in the upper panels, and blots for MeCP2 point mutation R133C are shown in the lower panels. Mock-treated (DMSO-treated) cells are shown for comparison. Total α-tubulin was measured as a loading control, along with β-actin.

Overall, the results indicate that the HDAC6 inhibitors, particularly QTX153, are capable of restoring wild-type levels of acetyl-α-tubulin in different genetic and differentiation contexts, without any associated toxicity. In particular, QTX153 demonstrated improved restoration of acetyl-α-tubulin levels compared to ACY1215 at similar concentrations, while also showing reduced toxicity.

### EXAMPLE 3: Ability of HDAC6 inhibitors to restore neurosphere formation defects in neural progenitor cells

A major feature of neural progenitor cells is their ability to grow in 3D spheres when detached from the growing surface. The size and shape of these neurospheres serve as indicators of the viability and pluripotent capacity of the cells. Divergence from the wild-type cells indicates an unhealthy cellular status and defects in differentiation. Rett syndrome (RTT) cells exhibit impaired neurosphere formation at the progenitor cell level.

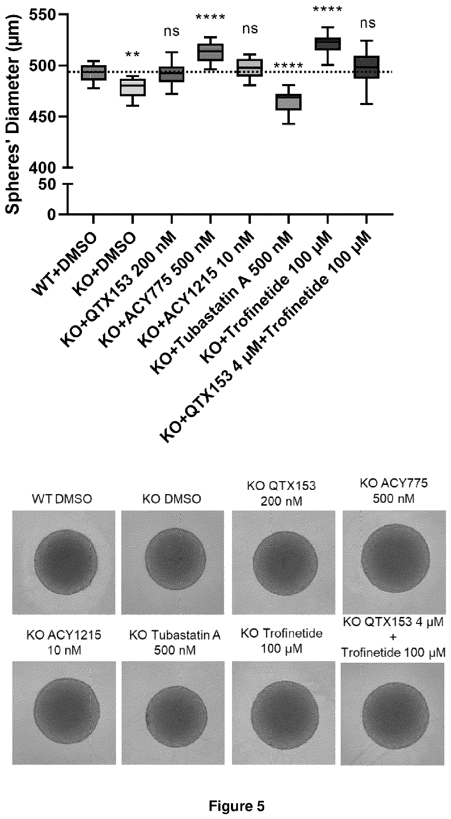

To assess the ability of HDAC6 inhibitors to rescue neurosphere formation defects, *MECP2-*KO neural progenitor cells were treated with QTX153, ACY775, ACY125, Tubustatin A, Trofinetide and QTX153 and Trofinetide under conditions that promote neurosphere formation, and the sphere diameter was quantified. The results are presented in Figure 5.

Neurosphere formation in *MECP2*-KO neural progenitor cells was analyzed upon treatment with the HDAC6 inhibitors (and Trofinetide) or with DMSO. Sphere diameter was quantified using ImageJ software (v1.53k) 7 days post-plating in non-adherent culture dishes (upper graph). The dotted line indicates the wildtype sphere diameter. Representative images of the spheres for each condition are shown in the photos below the graph in Figure 5. Ordinary one-way ANOVA was used for multiple comparisons relative to the wild-type (WT) condition (ns = not statistically significant; **, p < 0.01; ****, p < 0.0001).

Among the compounds tested, QTX153 (assayed at two different concentrations) and ACY1215 were able to restore the diameter of wild-type cells in *MECP2*-KO cells, whereas the other molecules displayed deviation from the wild-type mean. The combination of QTX153 and Trofinetide had an effect similar to QTX153 alone (see Figure 5).

### Example 4: Ability of HDAC6 inhibitors to restore neuronal morphology.

One of the hallmarks of Rett syndrome at the cellular and tissue level is the impaired maturation of neurons, which is associated with reduced complexity and a lower number of neurites and branching. The ability of HDAC6 inhibitors and Trofinetide to reverse this phenotype was tested.

Briefly: Control WT neural progenitors or *MECP2*-KO cells were driven towards glutamatergic differentiation for 7 days under mock-treatment (DMSO) or treatment with the indicated compounds. Cells were then stained with MAP2 and reconstructed *in silico* from confocal images using NeuroStudio software. Automatic analysis of the cells allowed for total branch points per each condition. All statistical comparisons were carried out relative to the *MECP2*-KO+DMSO cells. Ordinary one-way ANOVA was used for multiple comparisons relative to the WT condition. (*, p < 0.05; **, p < 0.01; ***, p < 0.001; ****, p < 0.0001, ns = not significant).

NeuroStudio analysis of reconstructed whole neurons in culture was carried out as follows, the three-dimensional dendritic network was constructed with a Sholl analysis by automated segmentation and represented by spheres/circles. The soma of the neurons is manually established, while neuronal components such as neurites, branching, and spines are automatically reconstructed.

Sholl analysis for quantitative estimation of neuronal components was carried out as follows: the algorithm considered the soma as the starting site, and concentric circles were drawn over a length of >20 µm with concentric circles of 0.5 µm throughout the projections (left). The number of branches or spines in each section was counted, and a three-dimensional reconstruction of the neuron was provided (right).

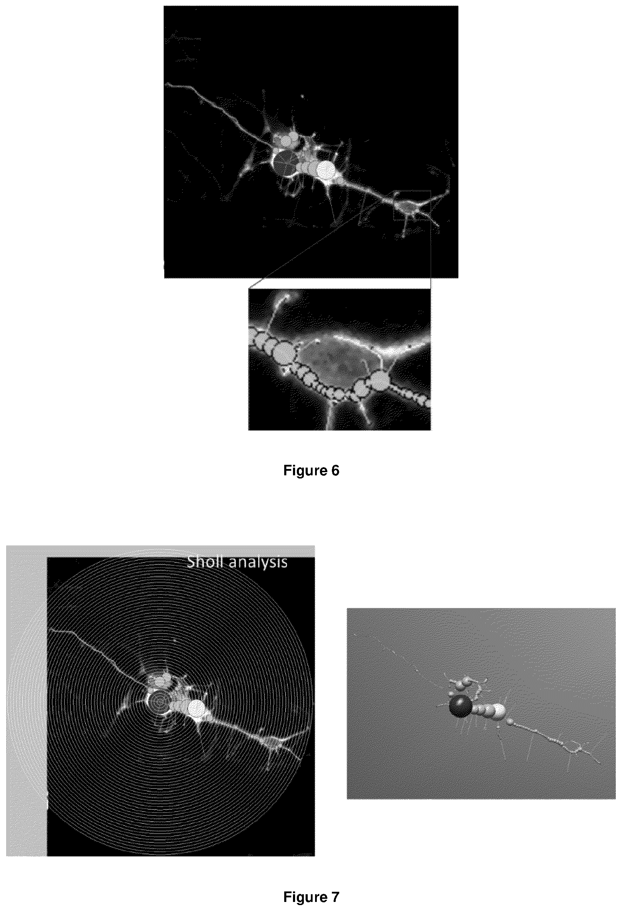

To achieve this, images of MAP2-stained neurons from differentiated cultures were acquired using confocal microscopy, and whole neurons were reconstructed using ImageJ software. Subsequently, the NeuroStudio program was used to track neuronal morphology and quantitatively estimate the number of branches (see Figures 6 and 7). This approach allows for a comprehensive analysis of neuronal morphology and provides insights into the potential of HDAC6 inhibitors and Trofinetide in restoring neuronal maturation in Rett syndrome.

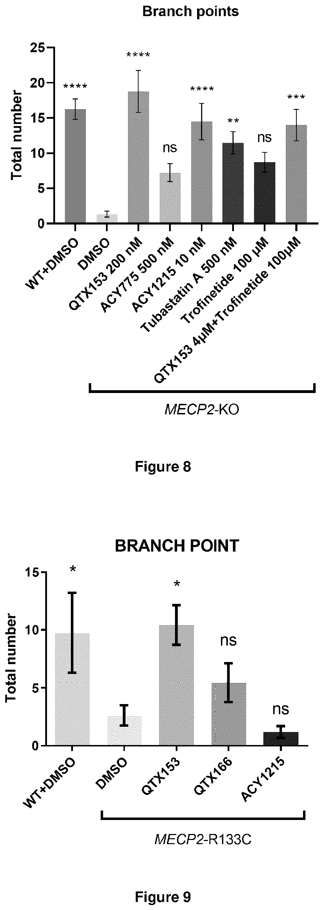

Figure 8 shows the information obtained from the analysis steps described above graphically. The data in Figure 8 show that QTX153 was able to rescue the morphological deficits of *MECP2*-KO neurons, restoring the number of branches to wildtype level. The other compounds tested, including ACY1215, Tubastatin A and Trofinetide, demonstrated some recovery in the number of branches, although to a lower extent than seen with QTX153.

These results suggest that the new HDAC6 inhibitors tested, particularly QTX153, are capable of rescuing morphological defects in a human cellular model of Rett syndrome.

To confirm these observations, QTX153 and other HDAC6 inhibitors were tested in the genetic background of the point mutant MECP2-R133C, which represents a common Rett syndrome mutation in patients.

Morphological Analysis of *MECP2-R133C* Neural Progenitors Under Glutamatergic Differentiation was carried out as follows: control WT neural progenitors or MECP2-R133C mutant cells were driven towards glutamatergic differentiation for 7 days under mock-treatment (DMSO) or treatment with the HDAC6 inhibitor QTX153. Cells were then stained with MAP2 and reconstructed *in silico* from confocal images with the NeuroStudio software. Automatic analysis of the cells allowed total branch points per each condition. Ordinary one-way ANOVA was used for multiple comparisons relative to the *MECP2*-R133C+DMSO condition. (*, p < 0.05; ns = not significant).

As shown in Figure 9, branching was significantly recovered upon treatment with QTX153, to a level comparable with the wildtype condition. Comparatively, ACY1215 showed no improvement in the number of branch points compared to treatment with DMSO.

This finding further supports the therapeutic potential of QTX153 in addressing the underlying cellular abnormalities associated with Rett syndrome.

### Example 5: In vivo assays in a male murine model; weight and survival curves

M*ecp2-*KO male were used as a murine model for RTT. To determine weigth gain and survival the mice were administered 160mg/kg of QTX153 from 4 weeks of age. Monitoring of the amelioration of symptoms was carried out. An initial pilot experiment with n=5 control and treated mice was conducted. Once no apparent toxicity was revealed and preliminary positive effects were observed (data not shown), the experiment was expanded to n=16 for each group. The following results refer to this larger experiment.

Animal weight serves as an indicator of general welfare, and significant weight loss compared to control may reflect the appearance of toxicity. To assess this aspect, mice in the two experimental groups were weighed daily (5 days per week) from the start of the experiments (at 4 weeks of age) until the endpoint (n=16). Five wild-type (WT) animals of the same age were weighed in parallel for comparison.

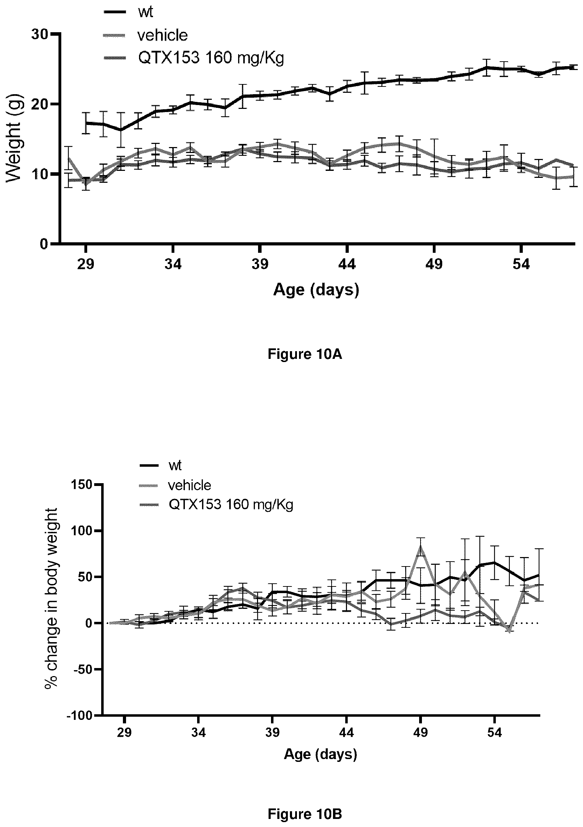

The body weight of control or treated mice over the course of the treatment of *MECP2*-KO male mice with QTX153 is represented in Figure 10. Weight in grams is shown from the start of the administration until the endpoint (Figure 10A). The same values are represented as a percentage of change relative to the body weight on the first day of administration (Figure 10B). No significant effect on body weight was observed following drug treatment in *MECP2-*KO mice. This suggests that the drug was well tolerated and induced no toxic effects at this dosage level. However, both vehicle-treated and QTX153-treated animals displayed reduced body weight compared to wild-type (WT) animals (n=16 for control, n=17 for QTX153-treated, and n=5 for WT animals).

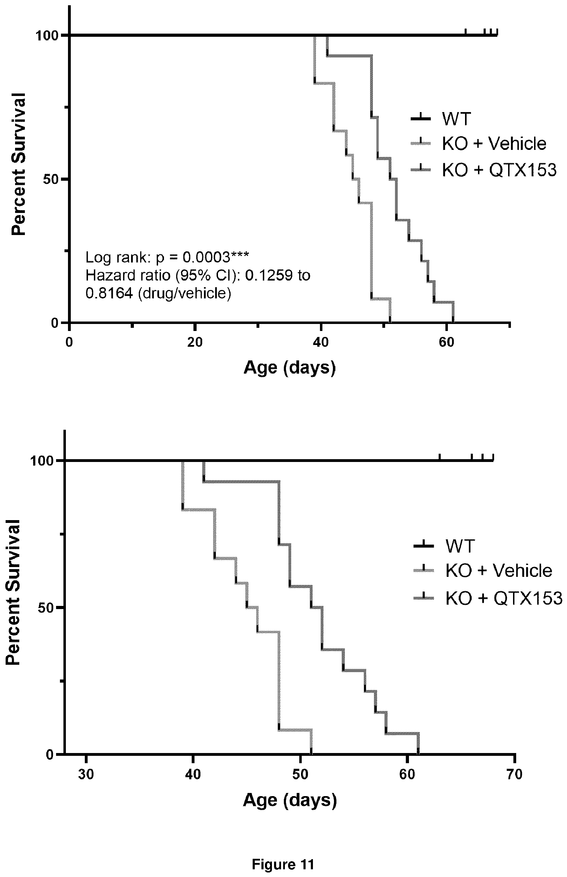

Next, the lifespan of the two experimental groups was evaluated by recording the endpoint date for each animal (see Figure 11). This was determined by the aggravation of symptoms to a degree that dictated their sacrifice, or by unexpected deaths, with exceptions made for animals sacrificed before the natural endpoint for sample collection and analysis.

The Kaplan-Meier method was used to estimate survival. A log-rank test (Mantel-Cox) was employed to compare experimental groups. Both graphs are equivalent, with the lower graph showing survival curves from the first day of treatment (day 28). Control Group: n = 12; QTX153 Group: n = 14. The median survival was 45.5 days for control mice and 51.5 days for drug-treated mice.

The graphs indicate that a longer lifespan was obtained for QTX153-treated mice (median survival: 45.5 days for vehicle-treated, 51.5 days for QTX153-treated). Hazard ratios from univariate Cox regressions were used to determine the association between treatment and overall survival. This analysis indicates that individuals in the treatment group (+QTX153) have a higher probability of survival over time compared to those in the reference group (+Vehicle). This observation suggests a potential beneficial effect of QTX153 treatment on the lifespan of *MECP2*-KO male mice

### Example 6: In vivo assays in a male murine model; phenotypic scores

M*ecp2-*KO mice develop a progression of symptoms compared to wild-type animals. These animals are typically normal until 4 weeks of age, after which they begin to experience cognitive and motor dysfunctions. The full manifestation of the Rett syndrome-like phenotype occurs at around 6 weeks of age, leading to rapid weight loss and death at approximately 10 weeks of age. However, the timing of these events can vary depending on the colony, with some colonies displaying earlier average death and others reaching older ages.

To investigate the efficacy of QTX153 in rescuing the Rett syndrome phenotype, the onset of symptoms was evaluated in the QTX153-treated group compared with the vehicle-treated group. Mobility, gait, tremor, breathing features, and hindlimb clasping were quantified to assess the alleviation of symptoms after drug administration. The average of each phenotypic score during each week of treatment was plotted and the average of the six scored symptoms was plotted to create a total score representing global phenotypic improvement.

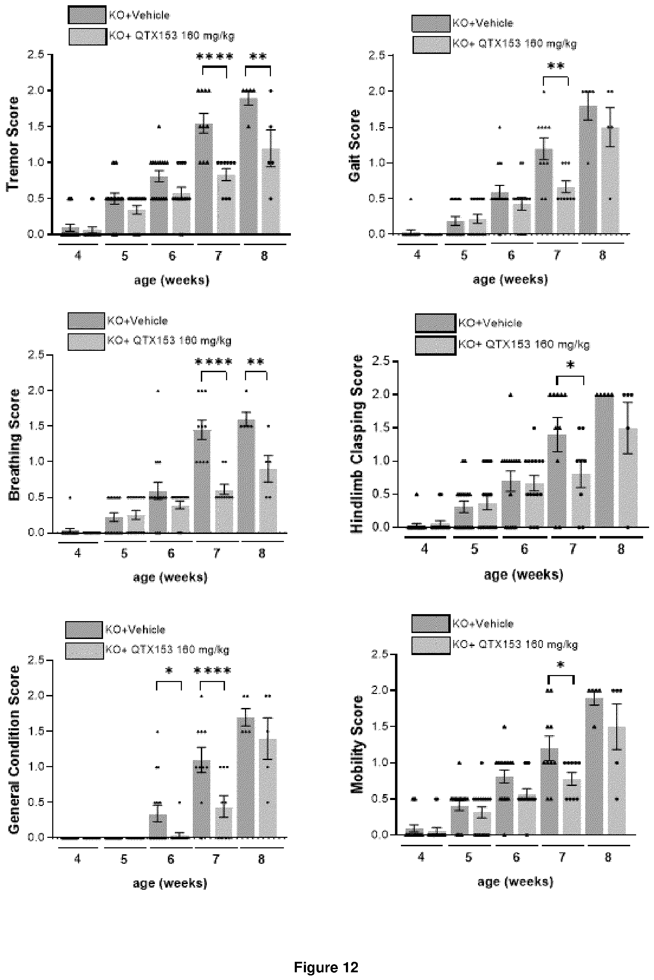

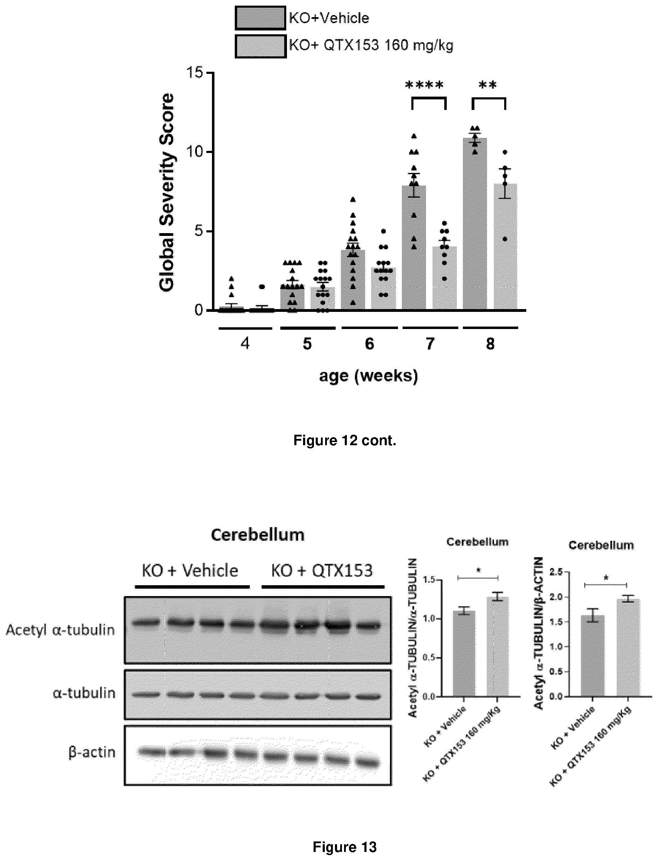

The results are shown in Figure 12 with each individual symptom represented on separate graphs on the right. Graphs represent mean ± SEM. For the described pairwise comparisons, ordinary one-way ANOVA was performed. Statistical significance is denoted as follows: *p < 0.05, **p < 0.01, and ****p < 0.0001

As shown in Figure 12, treatment with QTX153 at 160mg/kg improved the general well-being of the mice. The decrease in scores upon treatment corresponded to significant improvements in tremor, breathing, mobility, and gait phenotype, particularly at the 7th week of age. Hindlimb clasping was also improved, as well as the general condition of the mice. Overall, there was a significant amelioration of symptoms over weeks 7 and 8 of the animals' lives. This analysis highlights the potential therapeutic effect of QTX153 in mitigating the symptoms associated with Rett syndrome in M*ecp2-*KO mice.

### Example 7: In vivo assays in a male murine model; Molecular analysis of brain regions at endpoint

Given the ability of QTX153 to recover the Rett syndrome symptoms at the behavioural level, the molecular changes that might be elicited by QTX153 were then explored. Samples of the cerebellum were taken at the endpoint (at week 7 or 8) and analyzed by Western blot to detect potential changes in acetylation of α-tubulin, together with total levels of α-tubulin following treatment with QTX153. Figure 13 shows that levels of acetylated α-tubulin were significantly elevated in treated mice compared to vehicle-treated animals.

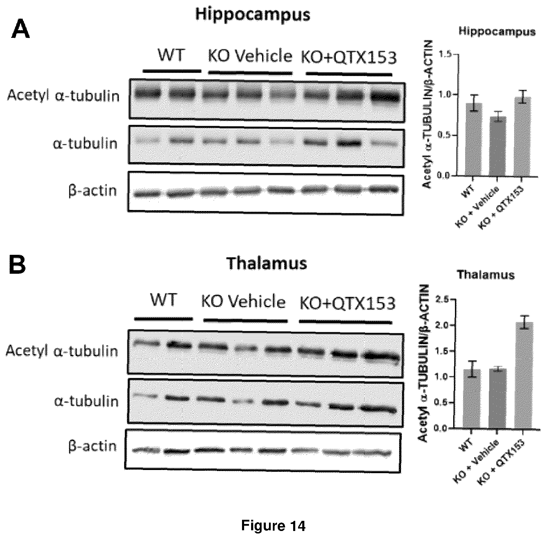

Levels of acetyl α-tubulin were assessed in other brain regions, as shown in Figure 14. An increase in the total levels of acetyl-α-tubulin was observed in the hippocampus and thalamus of QTX153-treated mice.

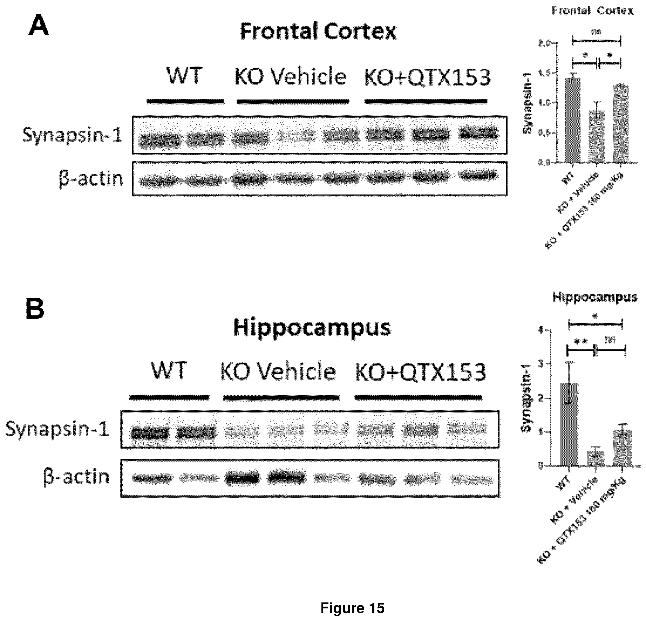

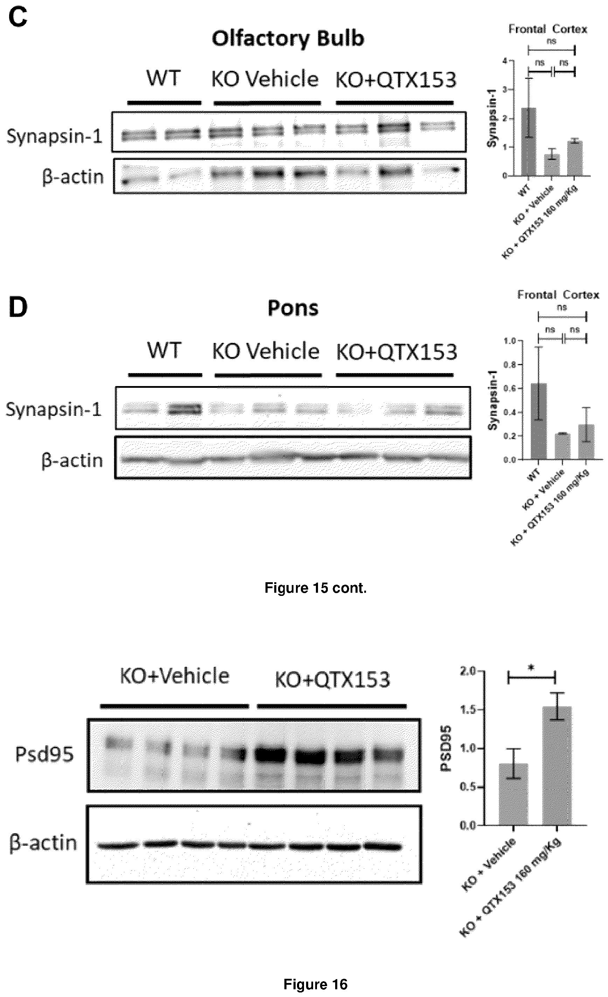

To further assess QTX153's ability to restore brain physiological activity, the expression levels of key synaptic markers was examined, including Synapsin-1 and PSD95 (markers of synaptic vesicles). In the case of Synapsin-1 (Figure 15), a partial recovery of its protein levels in the hippocampus and frontal cortex was observed.

The levels of the postsynaptic density protein 95 (Psd95, a key postsynaptic marker of excitatory neurons) were then assayed. Importantly, a sharp increase in Psd95 protein levels in the cerebellum of QTX153-treated mice was observed, as shown in Figure 16. This data suggests that synaptic transmission is partially restored by treatment with QTX153.

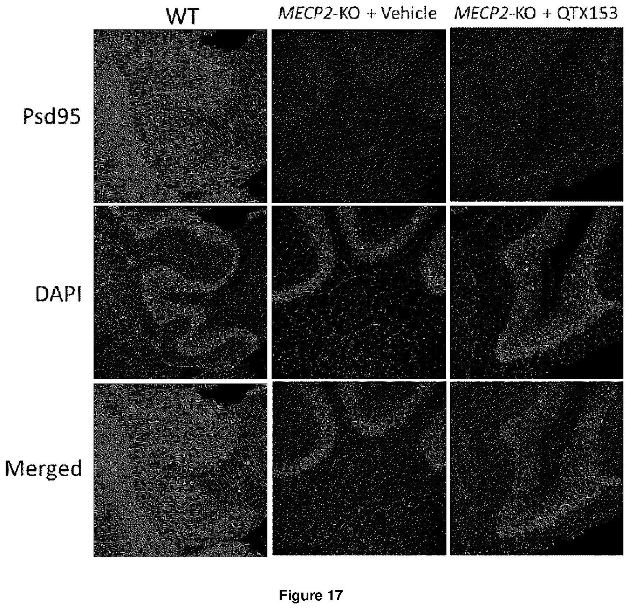

To confirm this effect, immunofluorescence staining was used to assess Psd95 levels in comparison to WT animals. As seen in Figure 17, Psd95 staining was almost absent in vehicle-treated mice but was moderately recovered (vs. Wild type) in QTX153-treated animals, suggesting that synaptic transmission is partially restored by QTX153. In light of this, excitatory neurons were further explored by analyzing the levels of VGlut1, a canonical marker of glutamatergic neurons (the most abundant excitatory neurons in the brain).

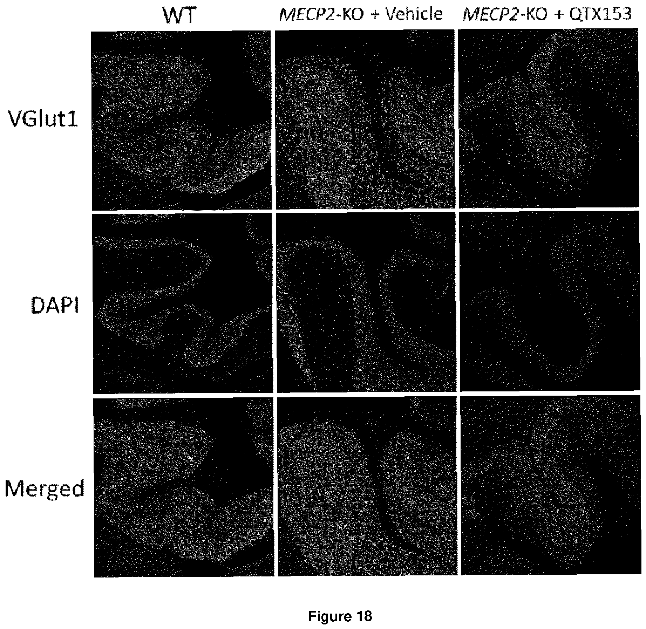

As depicted in Figure 18, levels of VGlut1 were abnormally high in the cerebellum of vehicle-treated Mecp2-KO mice, consistent with previous indications that Rett brains exhibit impaired excitatory/inhibitory balance. Conversely, mice administered with QTX153 showed levels similar to WT animals, indicating once again the ability of QTX153 to rescue synaptic connectivity.

### Example 8: In vivo assays in a male murine model; Morphological analysis of brain regions at endpoint

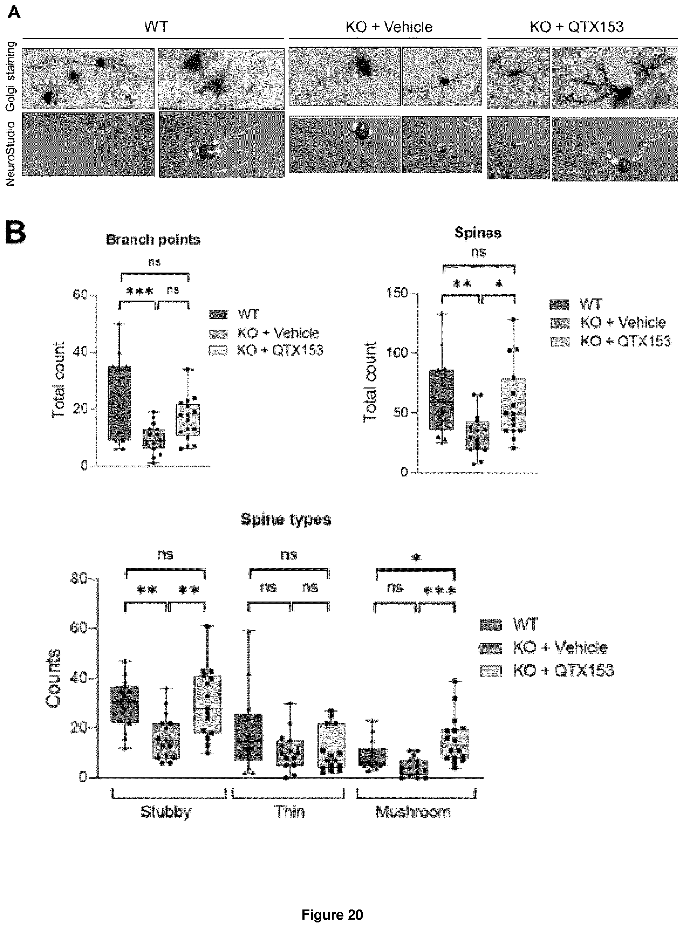

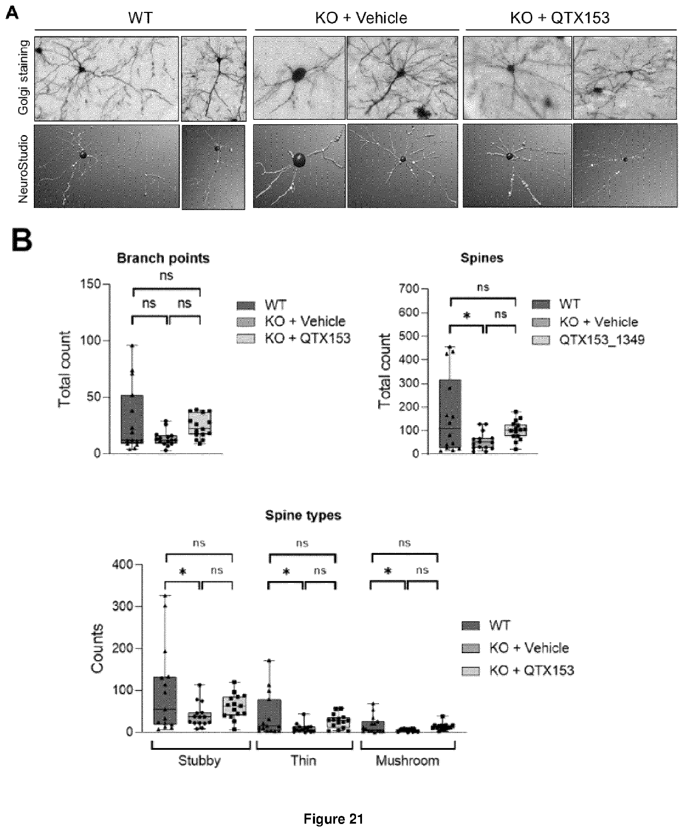

To assess the impact of QTX153 treatment on neuronal morphology, brain sections from WT, vehicle-treated, and QTX153-treated mice near the endpoint were subjected to whole neuronal impregnation via Golgi staining. Neuronal morphology, particularly dendritic arborization and spine density, reflects neuronal maturation and connectivity, with reduced complexity observed in Rett syndrome brains. Golgi staining was followed by digital morphological analysis of representative neurons from the experimental groups. Automatic analysis of the cells allowed quantification of total branch points and spine count for each condition. Regions analyzed included the hippocampus (Figure 19), cerebellum (Figure 20), and frontal cortex (Figure 21). Data in these figures, shows computational images of the neurons branching together with graphs comparing the number of branch points, the number of spines and the number of specific spine types.

### Conclusions

The results presented above show that QTX153 is able to restore key marker levels and neuronal morphology both *in vitro* and *in vivo,* indicating the therapeutic potential of this compound in the treatment of RTT. Additionally, QTX153 can rescue typical RTT symptoms *in vivo* without causing overt toxicity.

The viability assays demonstrated that the HDAC6 inhibitor QTX153 and the IGF1 analog Trofinetide exhibit minimal toxicity *in vitro,* with IC₅₀>10µM. Additionally, combining these compounds at higher concentrations maintains low toxicity. In contrast, ACY1215 displays higher toxicity than QTX153, with IC₅₀<1µM. Furthermore, QTX153 effectively restores physiological levels of acetyl-α-tubulin (at 200-400nM) in human cellular models of Rett syndrome, including full *MECP2*-KO and the point mutant MECP2-R133C cells, across progenitor and differentiating stages.

QTX153 effectively rescues impaired neurosphere formation by *MECP2*-KO neural progenitor cells, while other compounds produce abnormal sizes. Combination with Trofinetide yields similar results to QTX153 alone. In addition, deffects in neuronal morphology typical of RTT neurons are rescued by QTX153, in both MECP2-KO and MECP2-R133C neurons.

*In vivo* mouse results show that continued QTX153 treatment (160 mg/Kg) from 4 weeks of age in *Mecp2*-null male mice does not induce significant weight loss compared to controls but significantly prolongs lifespan, indicating a higher survival probability in treated mice. Phenotypic scores significantly improve in QTX153-treated M*ecp2-*KO animals at 7 weeks of age. Improvement occurs also in subsequent weeks, but the low number of surviving animals in control or treated groups limits statistical power. Furthermore, QTX153 treatment increases α-tubulin acetylation relative to total α-tubulin in the cerebellum of mice, with elevated levels of total acetyl-α-tubulin also in the hippocampus and thalamus. This suggests effective blood-brain-barrier penetration and activity in distant brain regions. Partial restoration of synaptic markers, such as Synapsin-1 and Psd95, is observed in specific brain regions of QTX153-treated mice, indicating synaptic connectivity improvement. Additionally, VGlut1 levels, indicative of glutamatergic neurons, are restored. Morphological analysis shows QTX153 ability to restore neuronal function and arborization in the hippocampus, cerebellum, and cortex of M*ecp2-*KO mice. The total number of spines is also partially recovered, with stubby spines showing robust recovery.

Overall, QTX153 exhibits low toxicity *in vitro* and *in vivo,* effectively restoring key features and symptoms of Rett syndrome in human cells and a murine model.

## Claims

1. A compound (HDAC6 inhibitor) for use in the treatment of Rett syndrome (RTT), wherein the compound has a formula according to formula (A): 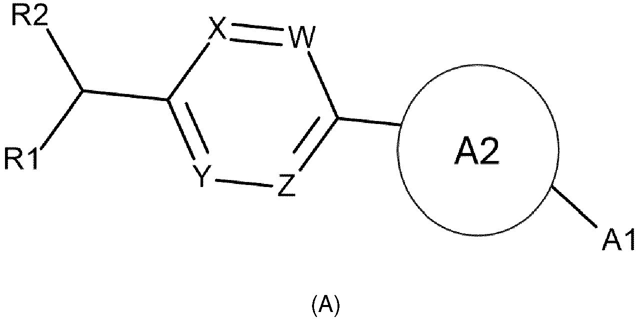 or a salt, solvate, stereoisomer or prodrug thereof,
wherein
A1 is CF₃, CHF_{2,} CH₂F
A2 is an oxadiazole ring;
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted aromatic or non-aromatic heterocyclic ring, wherein the ring comprises from 1 to 4 nitrogen atoms, and wherein it is one of these ring nitrogen atoms of the R₂ group that forms the bond to the rest of formula (A).

2. The compound for use according to claim 1, wherein the compound has a formula according to formula (B): 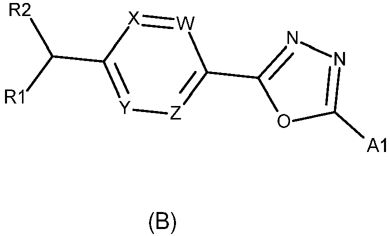
or a salt, solvate, stereoisomer or prodrug thereof,
wherein
A1 is CF₃, CHF_{2,} CH₂F
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted aromatic or non-aromatic heterocyclic ring, wherein the ring comprises from 1 to 4 nitrogen atoms, and wherein it is one of these ring nitrogen atoms of the R₂ group that forms the bond to the rest of formula (B);
preferably; wherein the compound has a formula according to formula (I): 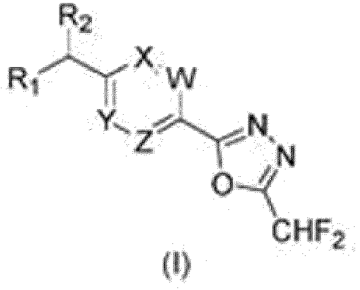
or a salt, solvate, stereoisomer or prodrug thereof,
wherein
one or two of W, X, Y or Z is N and the remainder of W, X, Y and Z are each CH, or each W, X, Y and Z is CH;
R₁ is H; unsubstituted or substituted alkyl; or halogen; and
R₂ is an unsubstituted or substituted aromatic or non-aromatic heterocyclic ring, wherein the ring comprises from 1 to 4 nitrogen atoms, and wherein it is one of these ring nitrogen atoms of the R₂ group that forms the bond to the rest of formula (B);
preferably, wherein the compound has a formula according to formula (II) 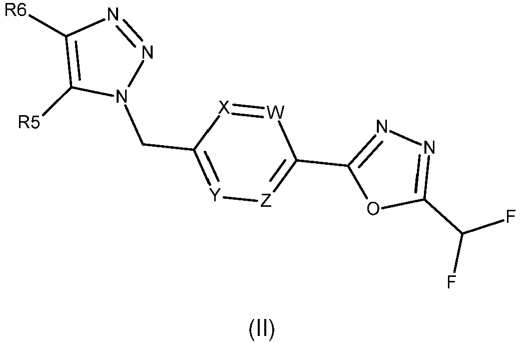
wherein
R5 is hydrogen or a substituted or unsubstituted C₁₋₆ alkyl,
R6 is a substituted or unsubstituted 5 to 8 membered aryl group or a substituted or unsubstituted 5 to 8 membered heteroaryl group
or wherein, R5 and R6 together with the carbons to which they are attached form a substituted or unsubstituted 5 to 8 membered aryl group or a substituted or unsubstituted 5 to 8 membered heteroaryl group.

3. The compound for use according to claim 2 or a salt, solvate, stereoisomer or prodrug thereof, wherein one of W, X, Y or Z is N and the remainder of W, X, Y and Z are each CH
and wherein R5 is hydrogen and R6 is a halo-substituted phenyl group; or
wherein R5 and R6 together with the carbons to which they are attached form a halo-substituted phenyl group
preferably wherein X or Y is N and the remainder of W, X, Y and Z are each CH.

4. The compound for use according to claim 2 or 3, wherein the compound has a formula according to formula (III) 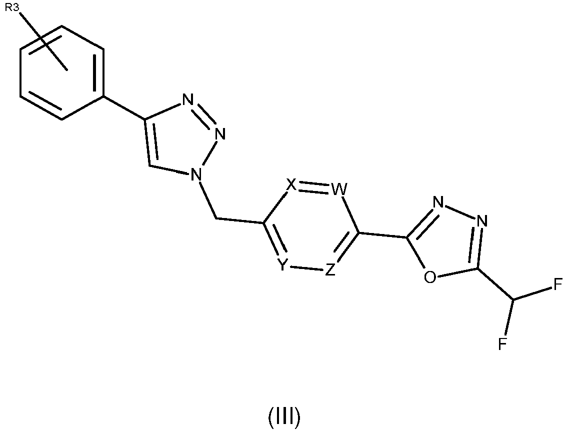
or a salt, solvate, stereoisomer or prodrug thereof;
wherein
one or two of W, X, Y or Z is N and the remainder of W, X, Y and Z are each CH, or each W, X, Y and Z is CH;
and wherein R3 is absent, halo, C₁₋₆ alkyl, C₁₋₆ haloalkyl, OH or NH₂;
preferably, wherein the compound has a formula according to formula (IV) 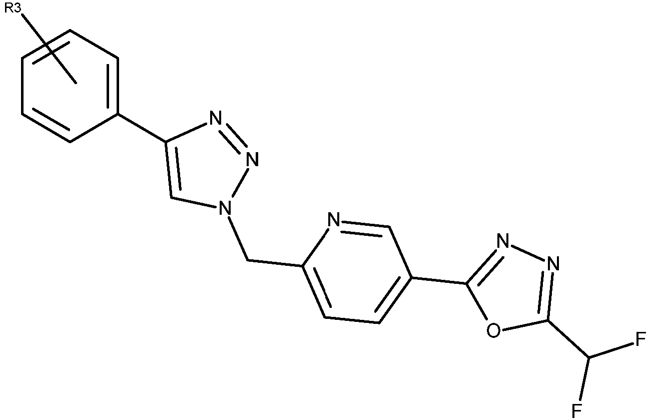 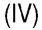
wherein R3 is halo.

5. The compound for use according to any one of the preceding claims, wherein the compound is: 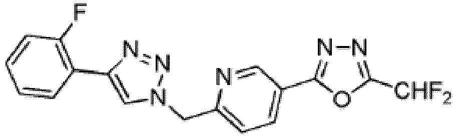 or a salt, solvate, stereoisomer or prodrug thereof.

6. The compound for use according to any one of claims 1 to 3, wherein the compound is: 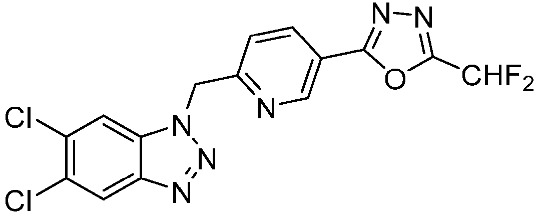 or a salt, solvate, stereoisomer or prodrug thereof.

7. The compound for use according to any one of the preceding claims, wherein the compound is administered simultaneously or sequentially with a second pharmaceutical agent.

8. The compound for use according to claim 7, wherein the second pharmaceutical agent is a neuropeptide analogue.

9. The compound for use according to claim 8, wherein the neuropeptide analogue is trofinetide.

10. The compound for use according to any one of the preceding claims, wherein the compound is administered at a dosage of at least 0.5 mg/kg, or at least 1 mg/kg, or at least 2 mg/kg, or at least 10 mg/kg .

11. The compound for use according to any one of the preceding claims, wherein the compound is administered orally, intravenously, intramuscularly, subcutaneously, sublingually, rectally, vaginally, nasally or by inhalation; preferably wherein the compound is administered orally, nasally or by inhalation.

12. The compound for use according to claim 11, wherein the compound is administered orally; preferably wherein the compound is administered as an oral tablet.

13. The compound for use according to any one of the preceding claims, wherein the compound is administered as part of a pharmaceutical composition comprising at least one excipient.

14. The compound for use according to any one of the preceding claims, wherein the compound is for use in preventing neurodegeneration and/or wherein the compound is for use in preventing loss of neuronal branch points.

15. The compound for use according to any one of the preceding claims, wherein the compound is for use in preventing tremor development and/or for stimulating an improvement in mobility.
